# EUROPEAN PATENT SPECIFICATION

(11) **EP 4 526 646 B1**
(45) Date of publication and mention of the grant of the patent: **12.11.2025**
(21) Application number: 23728284.3
(22) Date of filing: 17.05.2023
(51) Int. Cl.: G01N 15/14

(54) **METHOD AND APPARATUS FOR CLASSIFYING WHITE BLOOD CELLS**
VERFAHREN UND VORRICHTUNG ZUM KLASSIFIZIEREN WEISSER BLUTZELLEN
PROCÉDÉ ET APPAREIL DE CLASSIFICATION DE GLOBULES BLANCS

(30) Priority: 17.05.2022 EP 22173796
(43) Date of publication of application: 26.03.2025
(73) Proprietor: MEON Functional Diagnostics GmbH & Co KG, 8010 Graz (AT)
(72) Inventor: FLEMING, Alexander John, 8010 Graz (AT); ANISER, Wolfgang, 8051 Thal (AT)
(74) Representative: SONN Patentanwälte GmbH & Co KG
(86) International application number: PCT/EP2023/063240
(87) International publication number: WO 2023/222756

(56) References cited:
- WO-A1-02/057997
- WO-A2-2009/148549
- STEWART JUDITH C. ET AL: "Changes in fluorescence intensity of selected leukocyte surface markers following fixation", CYTOMETRY A, vol. 71A, no. 6, 1 January 2007 (2007-01-01), pages 379 - 385, XP055978900, ISSN: 1552-4922, DOI: 10.1002/cyto.a.20392

## Description

The present invention relates to a method and an apparatus for classifying white blood cells (WBCs).

In clinical diagnostics, the WBC count and differential is part of a complete blood analysis and an evaluation which is performed routinely. The WBC differential is essential at the point-of-care when screening for quantitative abnormalities in otherwise morphologically normal white blood cell populations, a condition that can occur in certain infectious diseases. Three-part WBC differentials identify and quantify lymphocytes, monocytes, and granulocytes and have been shown to be a reliable clinical benchmark when compared to conventional white blood cell differential counts.

WBC differentials may, e.g., be performed by an automated analyzer or manually, by examining blood smears under a microscope. In the automated differential, a blood sample is typically loaded onto an analyzer, which samples a small volume of blood and measures various properties of WBCs to produce a differential count. Typical hematology analyzers are based on the measurement of electrical parameters (such as impedance) or optical methods such as light scattering and cell staining in combination with digital image processing and flow cytometry. In the case of the manual differential, WBCs are usually counted on a stained microscope slide.

Numerous apparatuses and methods for classifying WBCs have been described in the prior art. WO 02/057997 discloses a method and arrangement for segmenting white blood cells in a digital colour image. EP 0 259 834 B1 discloses a method for classifying leukocytes with a flow cytometer by means of optical measurements on fluorochrome-stained blood cells. WO 2012/151105 A2 discloses systems and methods for analyzing blood samples, and more specifically for performing a basophil analysis. In one embodiment, the systems and methods include staining a blood sample with an exclusive cell membrane permeable fluorescent dye; and then using measurements of light scatter and fluorescence emission to distinguish basophils from other WBC sub-populations. WO 2014/143332 A1 discloses a method of performing a WBC analysis with an automated hematology analyzer, the method comprising diluting a sample of whole blood with a WBC analysis reagent comprising a membrane-permeable fluorescent dye and an osmolality adjustment component; and analyzing light scatter signals and a fluorescence emission signal from the sample as it traverses a flow cell.

The systems and methods disclosed in EP 0 259 834 B1, WO 2012/151105 A2 and WO 2014/143332 A1 include light scattering measurements using a laser as a light source in addition to fluorescence measurements, thus requiring complex and expensive optical measurement setups.

Forcucci et al. (Biomedical Optics Express 6.11 (2015): 4433-4446) discloses a low-cost, miniature achromatic microscope for identification of lymphocytes, monocytes, and granulocytes in samples of whole blood stained with acridine orange. The microscope was manufactured using rapid prototyping techniques of diamond turning and 3D printing and is intended for use at the point-of-care in low-resource settings. The measurement is based on the ratio of red to green fluorescence intensity. Powless et al. (Journal of Biomedical Optics 22.3 (2017): 035001) describes studies related to leukocyte differentials using different acridine orange staining and postprocessing methods in the context of an image-based point-of-care colorimetric cell classification scheme, wherein fluorescence intensity ratios are used.

The systems and methods disclosed in Forcucci et al. and Powless et al. are based on fluorescence intensity measurements only and allow the use of relatively low-cost optical setups. However, such methods are prone to errors and are typically limited with respect to reliability, accuracy and precision.

It is an object of the present invention to provide new methods and apparatuses for WBC classification that address at least some of the disadvantages of the prior art. In particular, it is an object of the invention to provide methods and apparatuses that are both low in cost and complexity and at the same time high in reliability, accuracy and precision.

Therefore, the present invention provides a method for classifying white blood cells (WBCs), the method comprising the steps of:
- providing a sample comprising a plurality of WBCs;
- adding at least one luminescent dye to the sample, thereby staining the WBCs;
- disposing the stained WBCs on a test surface;
- recording a luminescence image series comprising luminescence images of the stained WBCs on the test surface at a plurality of different timepoints, wherein each luminescence image comprises at least a first color channel and a second color channel;
- for each WBC of the plurality of WBCs, obtaining a luminescence data series by extracting from each luminescence image a luminescence datapoint associated with said WBC, wherein each luminescence datapoint comprises at least a first luminescence emission intensity value in the first color channel and a second luminescence emission intensity value in the second color channel; and
- analyzing the luminescence data series of each WBC in order to classify the WBC into one of at least two predetermined categories.

In a further aspect the invention provides an apparatus for classifying WBCs, preferably by the method according to the invention, the apparatus comprising:
- a sample holder for holding a test surface comprising a plurality of luminescently stained WBCs;
- a luminescence imaging system suitable for recording from the test surface luminescence images comprising at least a first color channel and a second color channel; and
- a processor configured to carry out the following steps:
   - obtaining a luminescence image series from the luminescence imaging system, the luminescence image series comprising luminescence images of the stained WBCs on the test surface at a plurality of different timepoints, wherein each luminescence image comprises at least a first color channel and a second color channel;
   - for each WBC of the plurality of WBCs, obtaining a luminescence data series by extracting from each luminescence image a luminescence datapoint associated with said WBC, wherein each luminescence datapoint comprises at least a first luminescence emission intensity value in the first color channel and a second luminescence emission intensity value in the second color channel; and
   - analyzing the luminescence data series of each WBC in order to classify the WBC into one of at least two predetermined categories.

The present invention provides an optical method for cell classification and cell counting of WBCs, which can be used in clinical diagnostics. The method is based on the analysis of luminescence images of stained blood cells. Different types of blood cells differ in structure and morphology; e.g., neutrophils are generally described as having a multilobed nucleus and a large portion of the cell is filled with cytoplasm, lymphocytes are generally described as having a spherical nucleus and a very small amount of cytoplasm, and monocytes are described with a kidney-shaped or single-lobed nucleus. Since the composition and environment within the nucleus differs from the cytoplasm, these morphological differences typically lead to differences in the luminescence signal, and the different types of WBCs can be distinguished using luminescence imaging.

In the context of the invention, it has been surprisingly found that the classification can be strongly improved by analyzing luminescence images of the stained blood cells at a plurality of different timepoints. The inventors observed that when WBCs are disposed on a surface for luminescence imaging, the luminescence signals obtained from the cells undergo changes over time that are characteristic for different types of WBCs.

Without being bound to a theory, the inventors speculate that a first reason for the observed differences is movement within the cells; i.e., rearrangement of cell organelles. In cells having a spherical nucleus (such as lymphocytes) the projected area of the nucleus on the test surface is less dependent on the orientation of the nucleus inside the cells; thus, the luminescence signal obtained from the nucleus is relatively constant over time. By contrast, in cells having a lobed nucleus (such as monocytes), the projected area on the test surface is strongly dependent on the orientation of the nucleus inside the cells and as a consequence, the luminescence signal varies as the orientation of the nucleus changes over time. At certain timepoints, the projection of the nucleus on the test surface may resemble that of a spherical nucleus; at other timepoints, it may appear completely different. Imaging the cell at a single timepoint may therefore lead to misidentification of the cell type. By taking luminescence images of the same cell at a plurality of timepoints, the classification becomes much more reliable.

A second reason for the observed differences in the luminescence signal over time may be movement of the cells as a whole; i.e., crawling or similar types of movements. Unlike erythrocytes, WBCs can attach to surfaces and begin to crawl along the surface in a random walk. The cytoplasm of WBCs can create prominences, which help cells actively crawl along surfaces and are commonly called pseudopods. As the different types of WBCs form pseudopods, their shape and by consequence the luminescence signal changes. In addition, cells that can form larger pseudopods can move in larger distance increments while crawling and therefore can potentially crawl faster than cells with smaller pseudopods. If the WBCs are imaged at a single timepoint, all information with respect to their movement is lost. By contrast, when multiple images of the same cell at different timepoints are analyzed, the movement of the cell over time can again be used to distinguish the cell types and improve classification.

All detailed descriptions and preferred embodiments disclosed herein for the method according to the invention also apply to the inventive apparatus. In particular, all preferred embodiments of the inventive method relating the measurement and analysis, especially to the recording of the luminescence image series, the obtaining of the luminescence data series, the analyzing of the luminescence data series, the clustering, the classifying and the counting of the WBCs, are also preferred in the context of the steps the processor of the apparatus is configured to carry out. Similarly, all preferred embodiments described with respect to the inventive apparatus are also preferred with respect to the inventive method.

Various different types of luminescence may be used in the context of the invention. It is preferred if the luminescence is fluorescence or phosphorescence, especially fluorescence. This applies to all features of the invention related to luminescence; e.g., when the luminescence is fluorescence, the luminescent dye is a fluorescent dye, the luminescence image series comprising luminescence images is a fluorescence image series comprising fluorescence images, the luminescence data series and luminescence datapoints are fluorescence data series and fluorescence datapoints, the luminescence emission intensity values are fluorescence emission intensity values, the luminescence imaging system is a fluorescence imaging system, etc. Thus, in the preferred embodiment, in which the luminescence is fluorescence, the inventive method comprises the steps of:
- providing a sample comprising a plurality of WBCs;
- adding at least one fluorescent dye to the sample, thereby staining the WBCs;
- disposing the stained WBCs on a test surface;
- recording a fluorescence image series comprising fluorescence images of the stained WBCs on the test surface at a plurality of different timepoints, wherein each fluorescence image comprises at least a first color channel and a second color channel;
- for each WBC of the plurality of WBCs, obtaining a fluorescence data series by extracting from each fluorescence image a fluorescence datapoint associated with said WBC, wherein each fluorescence datapoint comprises at least a first fluorescence emission intensity value in the first color channel and a second fluorescence emission intensity value in the second color channel; and
- analyzing the fluorescence data series of each WBC in order to classify the WBC into one of at least two predetermined categories.

Analyzing images taken at two different timepoints improves classification over a single image, for the reasons laid out above. However, classification is even further improved, when a larger number of images taken at different timepoints are analyzed since a larger number of images provides more information with respect to the changes in the luminescence signal over time. It is therefore preferred, if the luminescence image series comprises luminescence images at at least 2 different timepoints, preferably at least 3, more preferably at least 4, more preferably at least 5, more preferably at least 6, even more preferably at least 8, even more preferably at least 10, even more preferably at least 12, even more preferably at least 14, even more preferably at least 16, even more preferably at least 18, most preferably at least 20 different timepoints.

In the context of the invention it is further preferred, if the different timepoints are separated from each other by at least 1 second, preferably at least 5 seconds, preferably at least 10 seconds, more preferably at least 20 seconds, even more preferably at least 40 seconds, yet even more preferably at least 60 seconds, yet even more preferably by at least 90 seconds, most preferably by at least 120 seconds. Longer time intervals between the luminescence images allows to gather information over a longer time span and capturing more information with respect to the movement of the cells; both rearrangements inside the cells and movement of the cells on the test surface, which may be slow. Thus, it is also preferred if the different timepoints span a time period of at least 30 seconds, preferably at least 1 minute, more preferably at least 2 minutes, more preferably at least 4 minutes, more preferably at least 6 minutes, even more preferably at least 8 minutes, even more preferably at least 10 minutes, yet even more preferably at least 15 minutes, yet even more preferably at least 20 minutes, yet even more preferably at least 25 minutes, yet even more preferably at least 30 minutes, yet even more preferably at least 35 minutes, most preferably at least 40 minutes.

For instance, in a preferred embodiment, the luminescence data series may comprise at least 5 luminescence images recorded at different timepoints, each separated by at least 60 seconds. This embodiment would comprise a luminescence data series, in which exactly 5 luminescence images are recorded at different timepoints, wherein the time interval between each of the different timepoints is exactly 60 seconds. In this case, the timepoints would span a time period of exactly four minutes; i.e., images would be recorded at times t = 0 min, 1 min, 2 min, 3 min, and 4 min. However, this embodiment would not exclude that further luminescence images are recorded within the same time span; e.g., that multiple luminescence images are recorded at each timepoint or that additional timepoints are added within the time period. For instance, the embodiment defined by the luminescence data series comprising at least 5 luminescence images recorded at different timepoints, each separated by at least 60 seconds, would also comprise a luminescence data series comprising 9 luminescence images taken in intervals of 0.5 minutes; i.e., images would be recorded at times t = 0 min, 0.5 min, 1 min, 1.5 min, 2 min, 2.5 min, 3 min, 3.5 min, 4 min (which series comprises images taken at t = 0 min, 1 min, 2 min, 3 min, and 4 min).

In a preferred embodiment, the different timepoints span a time period of between 30 seconds and 300 minutes, preferably between 1 minute and 200 minutes, more preferably between 2 minutes and 150 minutes, more preferably between 4 minutes and 120 minutes, more preferably between 6 minutes and 105 minutes, even more preferably between 8 minutes and 90 minutes, even more preferably between 10 minutes and 75 minutes, yet even more preferably between 15 minutes and 70 minutes, yet even more preferably between 20 minutes and 65 minutes, yet even more preferably between 25 minutes and 60 minutes, yet even more preferably between 30 minutes and 55 minutes, yet even more preferably between 35 minutes and 50 minutes, most preferably between 40 minutes and 45 minutes. A longer time span allows capturing more information with respect to the movement of the cells; both rearrangements inside the cells and movement of the cells on the test surface.

The first color channel and the second color channel (and optionally further color channels) preferably correspond to different excitation or emission channels. For instance, a single excitation wavelength band may be used for excitation and the emission may be recorded in two different color channels, each corresponding to a different emission wavelength band. Alternatively, two different excitation wavelength bands may be used and the emission for excitation wavelength bands may be recorded at the same emission wavelength band or at different emission wavelength bands. In this case, the two color channels may correspond to two different excitation wavelength bands. However, it is preferred if the different color channels correspond to different emission wavelength bands. This allows using a single excitation light source and recording the signal from the different color channels simultaneously through different color filters; e.g., using a color camera.

It is preferred, if the first color channel and the second color channel correspond to color filters of a color filter array, preferably an RGB filter array, especially a Bayer filter. A color filter array is typically an array of color filters arranged on a grid of photosensors. A Bayer filter is a common type of color filter array, typically comprising RGB color filters. Such color filter arrays, especially Bayer filters, are commonly used in single-chip digital image sensors used in digital cameras to create color images. Such color filter arrays are particularly advantageous in the context of the inventive method and apparatus, since they are widely available at low cost and since they allow simultaneously recording images of the emission in two or more different color channels.

In a preferred embodiment, each luminescence image further comprises a third color channel. In this embodiment, each luminescence datapoint preferably further comprises at least a third luminescence emission intensity value in the third color channel. This allows to record even more information from the sample, which can lead to even better classification. For instance, relative changes in two color channels (e.g., the red and green channels of an RGB filter) may be used as a primary parameter for classifying the cells and the third color channel (e.g., the blue channel of the RGB filter) may be used for calibration and/or normalization.

In the context of the inventive method, it is preferred if the luminescence image series is recorded using an image sensor, preferably a CCD sensor or a CMOS sensor, especially a CMOS sensor. Similarly, in the context of the inventive apparatus it is preferred if the luminescence imaging system comprises an image sensor, preferably a CCD sensor or a CMOS sensor, especially a CMOS sensor. Both for the inventive method and apparatus it is particularly preferred, if the image sensor comprises a color filter array, preferably wherein the color filter array is an RGB filter array, preferably a Bayer filter. Such image sensor, especially with color filter arrays, are widely available at low costs and are particularly suitable for recording the image series in the context of the inventive method.

In a preferred embodiment, the recording of the luminescence image series comprises exciting the stained WBCs with an excitation light source, preferably wherein the excitation light source has a peak excitation wavelength between 300 nm and 700 nm, preferably between 350 nm and 600 nm, more preferably between 380 nm and 550 nm, even more preferably between 400 nm and 520 nm, most preferably between 420 nm and 500 nm.

In a further preferred embodiment, the first color channel is collected through a first band-pass filter, preferably wherein the first band-pass filter has a center wavelength between 350 nm and 750 nm, preferably 400 nm and 700 nm, more preferably between 450 nm and 650 nm, most preferably 480 nm and 600 nm. It is further preferred, if the second color channel is collected through a second band-pass filter, preferably wherein the second band-pass filter has a center wavelength between 450 nm and 850 nm, preferably 500 nm and 800 nm, more preferably between 550 nm and 750 nm, most preferably 580 nm and 700 nm. In an alternative embodiment, which is also preferred, the second color channel is collected through a long-pass filter, preferably wherein the long-pass filter has a cut-off wavelength between 400 nm and 800 nm, preferably 450 nm and 750 nm, more preferably between 500 nm and 700 nm, most preferably 530 nm and 650 nm. The first band-pass filter and/or the second band-pass filter preferably have a bandwidth between 1 nm and 300 nm, preferably between 5 nm and 250 nm, more preferably between 10 nm and 200 nm, most preferably between 20 nm and 150 nm.

The test surface on which the stained WBCs are disposed preferably is a transparent surface. This allows recording luminescence images from below; i.e., from the side of the surface that is opposite from the side on which the WBCs are disposed. In the context of the invention, "transparent" is preferably defined as the surface having a transmittance of at least 30 %, preferably at least 50%, especially at least 80%, in the wavelength range of 350 nm to 850 nm, preferably 450 nm to 700 nm, especially at 550 nm. However, the measurement may also be done from above; i.e., from the same side of the surface on which the WBCs are disposed. In this case, it is preferred if the surface is opaque, preferably defined as having a transmittance of less than 30 % in the above-mentioned wavelength ranges. However, in the context of the invention it is preferred if the measurement is done from below since this leads to reduced interferences from the sample; e.g., from absorption, light scattering, etc.

In the context of the inventive method, it is preferred if the WBCs disposed on the test surface adhere to said test surface. In this context, adhering preferably means that the cells are allowed to form an intimate contact with the surface and remain capable of lateral translation along the surface in any direction, so that they are able to crawl on the surface. Thus, preferably, the cell is allowed to crawl to the test surface. In this context, "crawling" is preferably understood as a process by which a cell can rearrange parts of the cell to extend pseudopods and propel the cell forward by active retraction of pseudopod, in a repetitive cyclical manner, to effectuate forward motion of the cell in any lateral direction along a surface.

In a preferred embodiment, the test surface is a plastic surface, preferably a polystyrene surface. Plastic, in particular polystyrene, is particularly well suited for allowing the WBCs to adhere and to crawl.

Adherence of the cells to the test surface can be achieved through sedimentation. In this context, pretreatment of a test surface can be used to further enhance attachment of the cells. It is therefore preferred, if the test surface is pretreated for cell attachment. Preferred materials and surface treatments are disclosed, e.g., in Shen and Horbett (J. Biomedical Material Research 57.3 (2001): 336-345), Ramsey et al. (In Vitro 20.10 (1984): 802-808), and Curtis et al. (The Journal of Cell Biology 97.5 (1983): 1500-1506).

The sample used in the context of the invention may be any type of sample comprising WBCs. For instance, the sample may consist of water or a suitable buffer comprising WBCs that have been previously isolated from a different source. However, it is preferred if the sample is a blood sample, preferably whole blood. The sample may be diluted or undiluted; preferably, the sample is diluted whole blood. Preferably the blood sample is obtained from a patient, preferably wherein the patient is an animal, preferably a mammal, especially a human. The inventive method may therefore also comprise the step of obtaining a blood sample from a patient.

Any type of luminescent dye may be used in the context of the inventive method. However, it is especially preferred that the at least one luminescent dye is cell-permeable. Using a cell-permeable luminescent dye has the advantage of obtaining luminescence signals from inside of the cells, making it possible to distinguish different types of cells also by their internal morphology and/or rearrangement of organelles. This is advantageous over a non-permeable luminescent dye, since the latter makes it necessary to primarily rely on the shape and movement of the cell for classification.

In the context of the invention, it is especially preferred if the at least one luminescent dye is suitable for live cell imaging. This has the advantage that the cells continue to move, both internally and externally, during the measurement, which provides for more information and therefore again better classification. Thus, preferably, the WBCs remain viable during the recording of the image series, preferably maintaining the internal cell rearrangements typical of living cells.

In a preferred embodiment, the at least one luminescent dye is a compartment and/or organelle specific dye, preferably a nuclear stain, especially a DNA stain. Different types of WBCs differ with respect to their internal morphology, especially with respect to the morphology of the nucleus. Thus, by specifically staining certain compartments or organelles, especially the nucleus, it is possible to distinguish different types of cells even more clearly.

In a further preferred embodiment, the at least one luminescent dye is a fluorogenic and/or solvatochromic luminescent dye. It is especially preferred, if the at least one luminescent dye comprises an environmentally sensitive luminophore, preferably fluorophore. It is particularly preferred, if the luminescent dye is a metachromatic luminescent dye. In such fluorogenic, solvatochromic, environmentally sensitive, and/or metachromatic luminophores the emission intensity and/or wavelength changes based on the microenvironment of the luminophore. Thus, the signal may change depending on the microenvironment in which the luminophore is located; e.g., inside specific cellular compartments.

It is particularly advantageous, if the excitation and/or emission spectrum, especially the emission spectrum, of the luminophore changes based on its microenvironment; especially, if said spectrum is different in different compartments of the cells. In this case, the relative intensities recorded in the different color channels of the luminescence images changes depending on the microenvironment of the luminophore. For example, different intensity ratios between the two color channels may be obtained from luminophore present in the nucleus vs. the cytosol. In a cell having a more spherical nucleus (such as a lymphocyte), the proportion of signal obtained from the cytoplasm and from the nucleus remains relatively constant over time and therefore the intensity ratio between the color channels remains relatively constant. By contrast, in a cell having an asymmetrically or non-spherically shaped nucleus (such as a monocyte), the proportion of the signal obtained from the cytoplasm and from the nucleus may change over time as the nucleus moves inside the cell and the surface area of its projection on the test surface changes. In this case, also the intensity ratio between the color channels may vary more strongly over time. In this way, valuable information with respect to the shape of the nucleus is obtained, which can be used for improving classification of the cells.

For these reasons, metachromatic and/or solvatochromic luminescent dyes are particularly preferred in the context of the invention. Solvatochromic luminescent dyes may preferably be defined as dyes that change color depending upon their microenvironment, especially the polarity of their microenvironment. Metachromatic dyes are preferably defined as dyes that change color when they bind to different elements of a cell or tissue. Most dyes are solvatochromic or metachromatic to some extent; thus, for most dyes analyzing the luminescence in two color channels lead to the above-mentioned advantages at least to some degree. However, even if a dye is used that is not solvatochromic or metachromatic at all, recording luminescence images comprising at least two color channels is advantageous, since comparing the two color channels allows to reduce noise or interference that may affect the luminescence signal over the time course of the luminescence data series.

In connection with all embodiments of the luminescent dye, it is preferred if the luminescent dye is a fluorescent dye or a phosphorescent dye, especially a fluorescent dye.

The at least one luminescent dye is preferably selected from the group consisting of acridine orange, hexidium iodide, SYBR 11, SYBR Green series dye, SYTO RNA Select, SYTO 11, SYTO 12, SYTO 13, SYTO 14, SYTO 16, SYTO 21, SYTO 24, and/or SYTO 25. Acridine orange (AO) is particularly preferred. AO is a metachromatic, cell permeable, fluorescent dye for staining nucleic acids and that is differently selective to single strand and double strand nucleic acids. For this reason, when AO is taken up into the blood cells it shows markedly different fluorescence properties in the different areas of the cell; especially in the nucleus (where most of the double stranded nucleic acids are located) and the cytoplasm, where the dye produces different emission colors. By monitoring the emission color (i.e., the relative fluorescence intensity in the at least two color channels), the internal movement of cell components can be monitored. The emission colors can conveniently be distinguished, e.g., by using regular RGB-color filters; e.g., a Bayer sensor.

In a preferred embodiment, the at least one luminescent dye, preferably AO, is added to the sample in a concentration between 0.01 to 10 µg/mL, preferably 0.05 to 2 µg/mL, most preferably 0.1 to 1 µg/mL. This concentration range provides a strong signal without affecting viability and/or movement of the cells too much.

In a further preferred embodiment of the invention, at least a first and a second luminescent dye are added to the sample, thereby staining the WBCs. All preferred embodiments described herein with respect to the at least one luminescent dye are equally preferred, individually, for both the first and second luminescent dye.

Preferably, the first luminescent dye differs from the second luminescent dye at least in its excitation and/or emission spectrum. In particular, it is preferred if the first luminescent dye has a different peak emission wavelength than the second luminescent dye. Preferably, the peak emission wavelength of the first luminescent dye and the peak emission wavelength of the second luminescent dye differ by at least 10 nm, preferably at least 20 nm, more preferably at least 40 nm, even more preferably at least 60 nm, most preferably at least 80 nm. It is further preferred, if the first luminescent dye and the second luminescent dye have different compartment and/or organelle specificities. This allows to improve classification in a similar way as described above in the context of solvatochromic and metachromatic dyes.

In the context of the invention, each WBC is preferably classified by comparing the luminescence data series obtained for said WBC to a predetermined reference. Any suitable method and reference may be used for such comparison. In the context of the invention, particularly advantageous methods for analyzing the luminescence data series have been found, which are described in more detail below. However, the invention is not limited to any specific method of analysis.

For instance, the predetermined reference may simply consist of ranges of luminescence intensities in the different color channels, that are each assigned to one of the predetermined categories. Alternatively, the reference may simply consist of ranges of luminescence intensity ratios that are each assigned to one of the predetermined categories. For instance, when a Bayer sensor is used, the ratio of the time-averaged mean intensity of the WBC in the red channel and the time-averaged mean intensity of the WBC in the blue channel may be determined, and the pre-determined reference may simply be a cut-off value above which the WBC is assigned to a first category and below which the WBC is assigned to a second category.

Preferably, the pre-determined reference is determined by calibration. For instance, the same steps as defined in the inventive method may be carried out with a reference sample or a number of many different samples, for which the WBCs have been classified using a reference method.

In a preferred embodiment, analyzing the luminescence data series of each WBC comprises determining a trajectory over time of the WBC in a color space formed by said color channels. For each WBC at each timepoint, it is possible to determine a coordinate in said color space, wherein the coordinate is defined by the luminescence intensity values in the color channels making up said color space. These luminescence intensity values may be the average luminescence intensity value measured for the WBC in the respective color channel. For example, a red-green color space may have a coordinate defined by the red color component value and the green color component value, of the red and green channel, respectively. When the luminescence images only contain two color channels, the color space may be a two-dimensional color space. When the luminescence images contain three or more color channels, the color space may be a three- or more-dimensional color space.

In order to determine the trajectory of the WBC over time, standard particle tracking algorithms may be used in order to identify the WBC in each successive luminescence image. Such a particle tracking algorithm typically is a method of examining a sequence of binary images to locate the same object as it moves spatially across the field-of-view of a camera over time in an image sequence, assign it a trajectory number and logically connect the region-of-interest (ROI) for this object in each frame to the same trajectory number. A ROI typically is a region defined in an image that is to be processed together and equally; for instance, the outline of a cell in a segmented image may define a ROI for that cell. Suitable particle tracking algorithms are known in the art and available to the skilled person; see e.g. Sbalzarini and Koumoutsakos (Journal of Structural Biology 151.2 (2005): 182-195).

In a preferred embodiment of the invention, analyzing the luminescence data series of each WBC further comprises determining a Centroid-Of-Trajectory (COT) for the WBC, wherein the COT corresponds to the centroid of the trajectory. A centroid is preferably defined as the arithmetic mean position of all points in a plane figure. If a physical object has uniform density, its center of mass is the same as the centroid of its shape. The COT is preferably defined as the coordinate in color space where the centroid of a trajectory is located. In place of the COT it is also possible to use the trajectory average, which is preferably defined as the ensemble average of all datapoints belonging to a trajectory, described as a single coordinate in color space corresponding to the centroid of the path shape and equivalent to the COT.

In a preferred embodiment of the invention, analyzing the luminescence data series of each WBC further comprises assigning the COT to one of the predetermined categories in order to classify the WBC. Preferably, analyzing the luminescence data series of each WBC further comprises assigning the COT to an area of a predetermined color space map comprising a plurality of areas, each area corresponding to one of the predetermined categories, thereby classifying the WBC into the category corresponding to said area.

Similar steps may be carried out in the context of a calibration in order to determine the pre-determined reference. Luminescence image series of a representative sample or a number of many different samples may be captured, preferably, wherein these samples are or have been also analyzed using reference methods. Then COTs from the WBCs contained in these samples may be generated from the images. These COTs may be clustered, each cluster representing an area in a color space map. Each cluster area on the color space map may be assigned one of the pre-determined categories, preferably a cell type. This (factory) pre-calibrated color space map with the assignment of cluster areas to different categories can then be used as the pre-determined reference for assigning the COT obtained for each WBC to one of categories by comparing its coordinates with the coordinates of the cluster areas of the pre-calibrated color space map. Then, the cell population of each cluster area may be determined by summing up the COTs of the individual cluster areas, and from this, the number of WBCs in each category (e.g., the categories being neutrophils, lymphocytes and monocytes/others) may be determined.

A cluster typically describes a group of data points in the color space that have more similarity with all other data points of the same cluster as when compared to datapoints outside the cluster. Many methods for clustering data are known in the art and are suitable in the context of the invention. For instance, k-means clustering may be used. K-means clustering is typically described as a method of assigning datapoints to a cluster based on the convergence of the sum of least squared Euclidean distances of datapoints to centroids for each cluster.

Clustering is advantageous not only in the context of determining the pre-determined reference but also during the measurement of the sample itself. In a preferred embodiment, the method thus further comprises the step of clustering the COTs of the WBCs into a plurality of clusters. Preferably the COTs of the WBCs are clustered into at least 2, preferably at least 3, more preferably at least 4, even more preferably at least 5, yet even more preferably at least 6, most preferably at least 7 cluster. Any suitable clustering method may be used; preferably, the clustering is by k-means clustering. Clustering the COTs of the WBCs in the sample allows assigning the WBCs to the predetermined categories as a group rather than individually, which leads to even more reliable classification; this is because WBCs are also compared to each other within the sample and classification is therefore less affected by variations between samples and between measurements.

Preferably, the method further comprises the step of determining for each cluster a Centroid-Of-Cluster (COC), wherein the COC corresponds to the centroid of the cluster. The COC may thus be defined as the unique coordinate in color space where the centroid of a cluster is located. Determining the COC allows to compare said COC to the pre-determined reference and thus to assign the cluster to one of the pre-determined categories as a whole. Thus, in a preferred embodiment, the method further comprises the step of assigning each cluster to one of the predetermined categories, thereby classifying the WBCs contained in said cluster into said category. Preferably each cluster is assigned to an area of a predetermined color space map comprising a plurality of areas, each area corresponding to one of the pre-determined categories, thereby classifying the WBCs contained in said cluster into the category corresponding to said area.

In a preferred embodiment, the method further comprises the step of determining a Centroid-Of-All-Clusters (COAC), wherein the COAC corresponds to the centroid of the COCs. The COAC may thus be defined as the unique coordinate location in color space where the centroid-of-all-clusters is located. Determining the COAC allows to determine a reference coordinate for the sample as a whole, which allows aligning the color space of the sample to a color space of a reference measurement; e.g., the pre-determined reference.

As an example, in order to determine the pre-determined reference, a calibration may be performed, whereby a luminescence image series of a number n of different samples may be acquired. Then, for each of the n samples, k Centroids-of-Clusters (COCs) in color space may be generated by clustering the COTs (e.g., by a k-means algorithm) with a predetermined number k of clusters, each of the k clusters representing a pre-determined category, preferably a cell type (wherein multiple clusters can also represent the same category; i.e., there may be more clusters than categories). The resulting n x k COCs may be located within k distinguishable regions on a color space map, each region containing a group of COCs in the form of a pile of n points. The envelopes of piles can be determined empirically or by mathematical methods. All envelopes together then enclose k regions, to each of which may be assigned a category and within which the COCs of the samples to be analyzed at the user side may be located. The envelopes may enclose one or more of the k regions. For the measurement of the sample, a luminescence image series may be acquired from the sample to be analyzed, the COTs of the sample to be analyzed may be clustered (e.g., again by a k-means algorithm) into a pre-determined number k of clusters, each of which is then assigned the category of the region of the pre-calibrated color space map that matches the coordinates of the respective COC.

The pre-calibrated color space map with registered COC regions with category labels can be adapted to the user's color space map by scaling the map axes. This has the advantage of reducing the effect of differences in the instrumental setup or in differences in the behavior of the blood cells investigated between calibration and measurement. The determination of the cell population may then be done by summing up the COTs belonging to each labelled COC region and from this the number of WBCs in each category; e.g., the number of neutrophils, lymphocytes and monocytes/others, can be determined.

In addition, during calibration, an average of the Centre-of-All-Clusters (COAC) for all samples may be determined. In this case, the pre-calibrated color space map with registered COC regions with category labels can be adapted to the user's color space map by aligning the COAC of the pre-calibrated color space map with the COAC of the user's color space map. This makes it possible to align the color space of the measurement to the color space of the pre-determined reference and thus allows an even better classification.

Thus, in a preferred embodiment of the invention, the method further comprises the step of creating a calibrated color space map from a predetermined color space map comprising a plurality of areas, each area corresponding to one of the predetermined categories, preferably by scaling the predetermined color space map to the COCs and/or by aligning the predetermined color space map with the COAC.

In a preferred embodiment, the analyzing the luminescence data series of each WBC further comprises assigning the COT to an area of the calibrated color space map, thereby classifying the WBC into the category corresponding to said area.

In a further preferred embodiment, the method further comprises the step of assigning each cluster to an area of the calibrated color space map, thereby classifying the WBCs contained in said cluster into the category corresponding to said area.

In yet a further preferred embodiment, the method further comprises the step of assigning each COC to an area of the calibrated color space map, thereby classifying the WBCs contained in the cluster corresponding to said COC into the category corresponding to said area.

In the context of the invention, it is preferred if the at least two predetermined categories are selected from the group consisting of granulocytes, lymphocytes, and monocytes; preferably granulocytes and lymphocytes. In a further preferred embodiment, the at least two predetermined categories are selected from the group consisting of neutrophils, lymphocytes, monocytes, basophils, and eosinophils; preferably neutrophils, lymphocytes and monocytes; especially neutrophils and lymphocytes. It is particularly preferred, if the WBCs are classified into one of at least three predetermined categories, preferably granulocytes, lymphocytes, and monocytes. It is especially preferred, if the WBCs are classified into one of at least three predetermined categories, preferably selected from the group consisting of neutrophils, lymphocytes, monocytes, basophils, and eosinophils; especially neutrophils, lymphocytes and monocytes. In a further preferred embodiment, the WBCs are classified into one of at least five predetermined categories, preferably neutrophils, lymphocytes, monocytes, basophils, and eosinophils.

In a preferred embodiment, the method further comprises the step of counting the number of WBCs in each of the predetermined categories. In a further preferred embodiment, the method further comprises the step of determining the proportion of WBCs in each of the predetermined categories.

Preferably the inventive method is for obtaining a WBC differential, preferably a 3-part WBC differential or a 5-part WBC differential, especially a 3-part WBC differential.

The present invention is further illustrated by the following figures and examples, without being limited thereto.

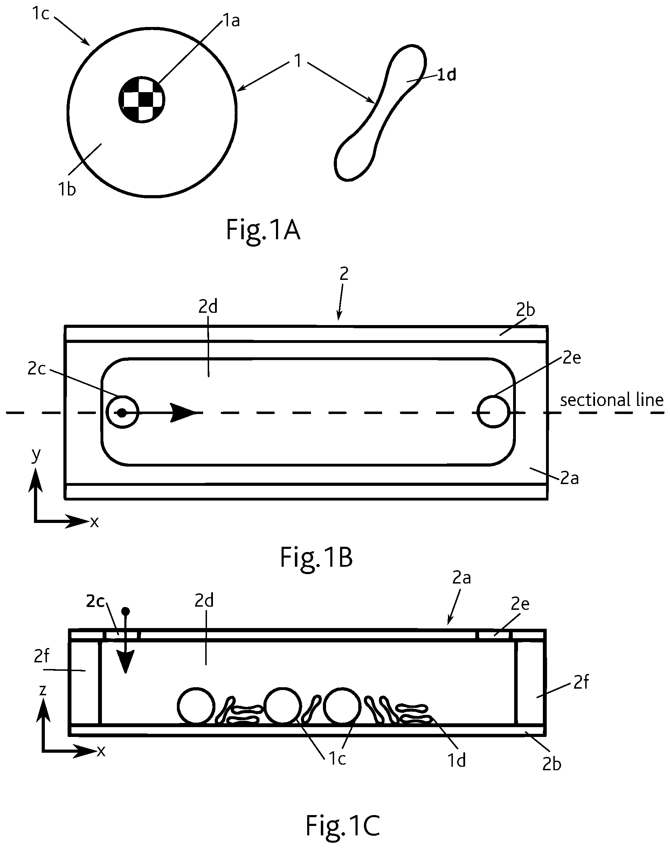

**Figure 1****:** Shows A) various blood cells, B) top view of a measurement chamber 2d (arrow indicates direction of sample flow in 2d), and C) side-view (through sectional line) of measurement chamber 2d with red and white blood cells sedimented on bottom surface 2b (shown here at monolayer surface coverage).

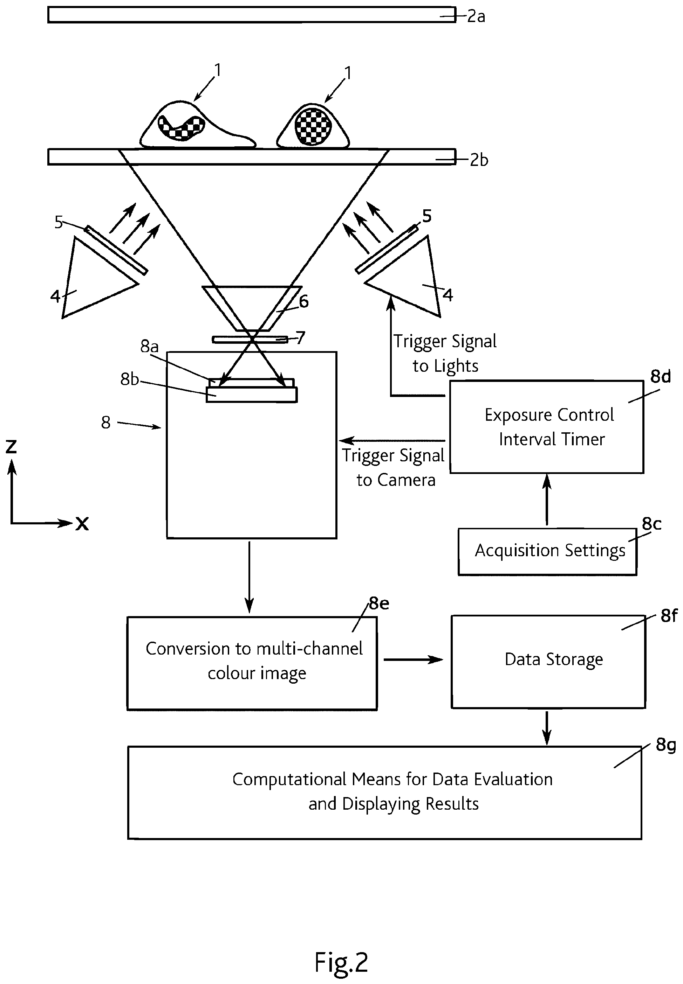

**Figure 2****:** Schematic diagram of an exemplary arrangement of instrument, camera, lens, lights, and filters to perform luminescence imaging of blood cells.

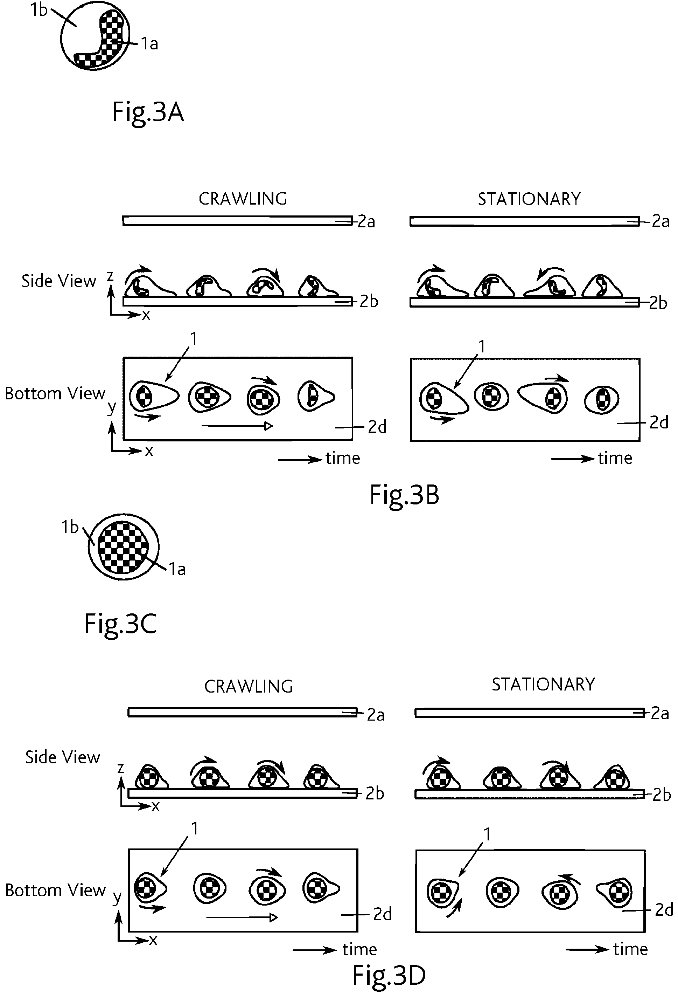

**Figure 3****:** A) Simplified cell structure of a Neutrophil, B) Side and bottom view perspectives showing the relative rearrangements of 1a and 1b cell components that can occur during cell crawling in the x-direction. Also shown are the Neutrophils that remain stationary despite attempts to crawl in various directions, C) Simplified cell structure of a Lymphocyte, D) Side and bottom view perspective showing the crawling action in the x-direction and Lymphocytes that remain effectively stationary despite attempts to crawl in various directions. Lymphocytes, when compared to Neutrophils, exhibit much less cell component 1a and 1b rearrangements with time.

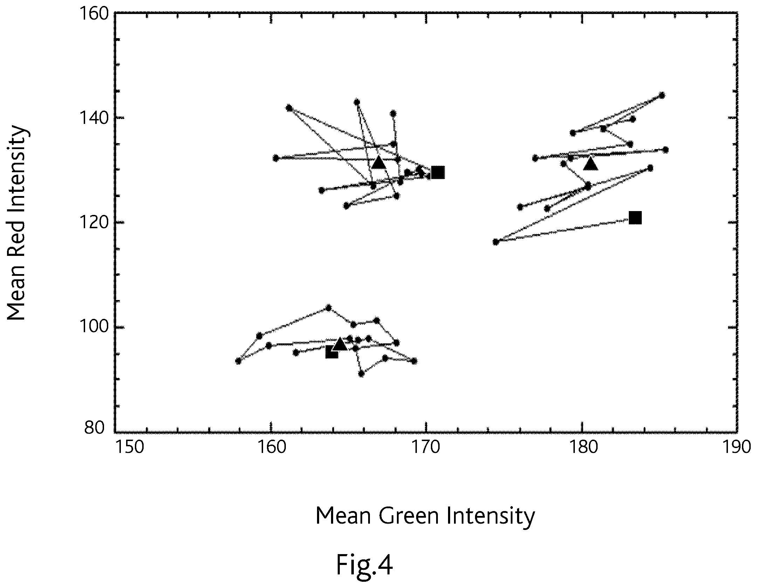

**Figure 4****:** Example scatter plot of the trajectories in the red-green color space of three example cells over time. Each data point of a trajectory represents one fluorescence data point obtained from one fluorescence image in the acquired fluorescence image series. Legend: Color space coordinates for a cell at frame n that belongs to trajectory m (•); color space coordinate for a cell at frame 1 that belongs to trajectory m (■); Centroid-of-Trajectory of trajectory m in color space (A).

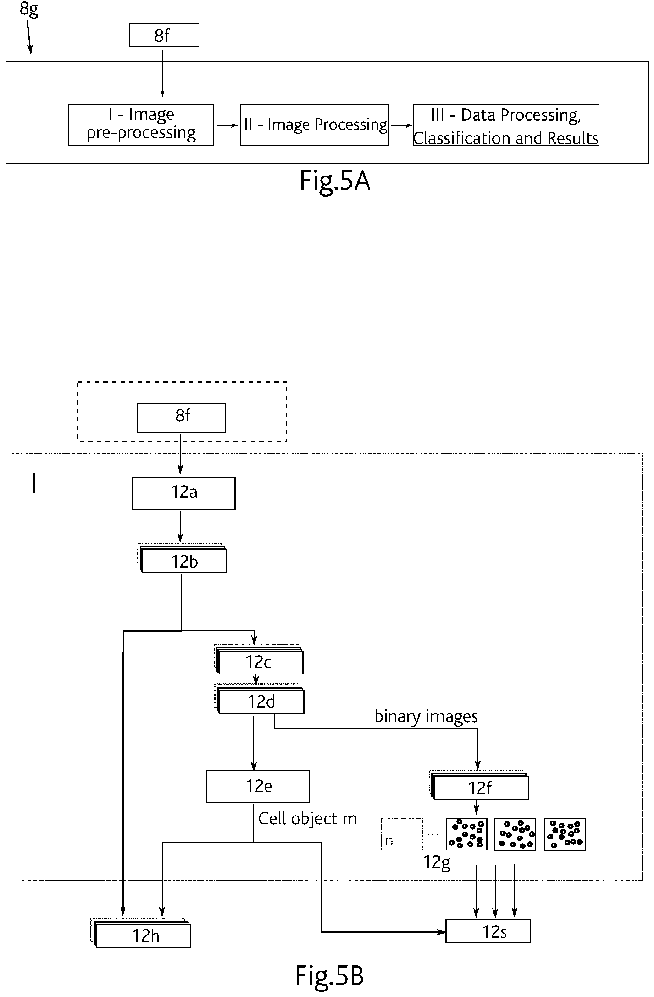

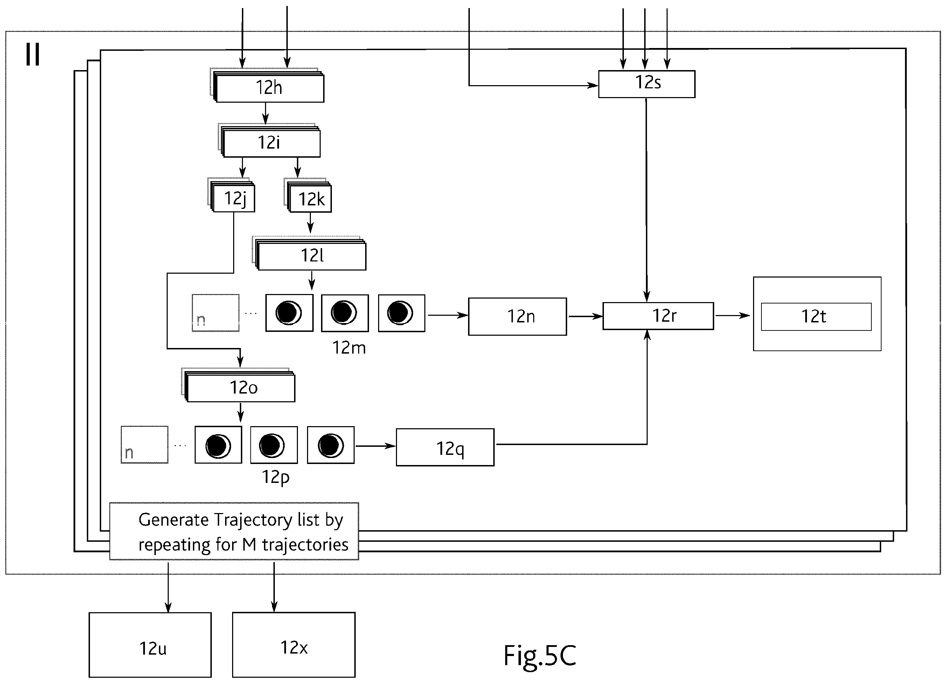

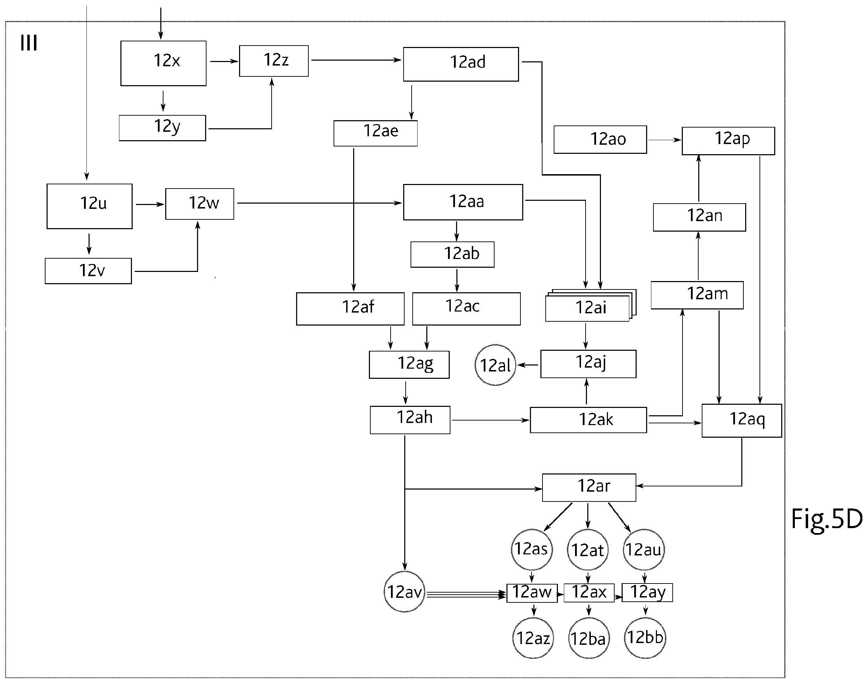

**Figure 5****:** Exemplary embodiment of an algorithm to classify images to obtain a 3-part differential cell count (DCC). A) Flowchart showing each part of the algorithm. B) Part I of the software steps used to preprocess images. C) Part II of the software steps used to process all images in the acquired image sequence that have been pre-processed in Part I. D) Part III of the software steps used to classify images, processed by Part II of the algorithm, and provide a final 3-part DCC result.

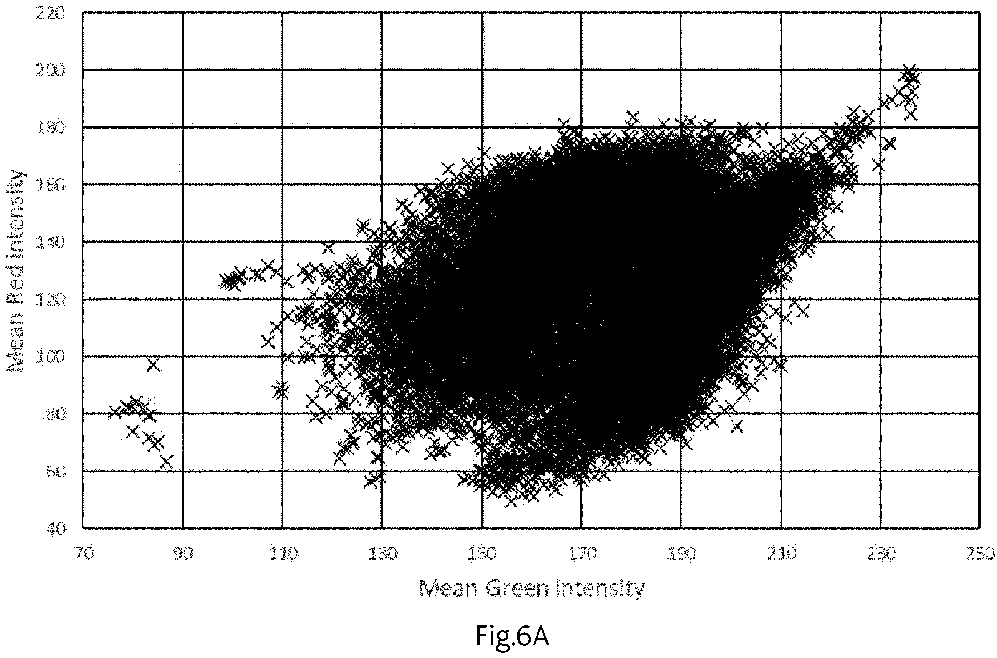

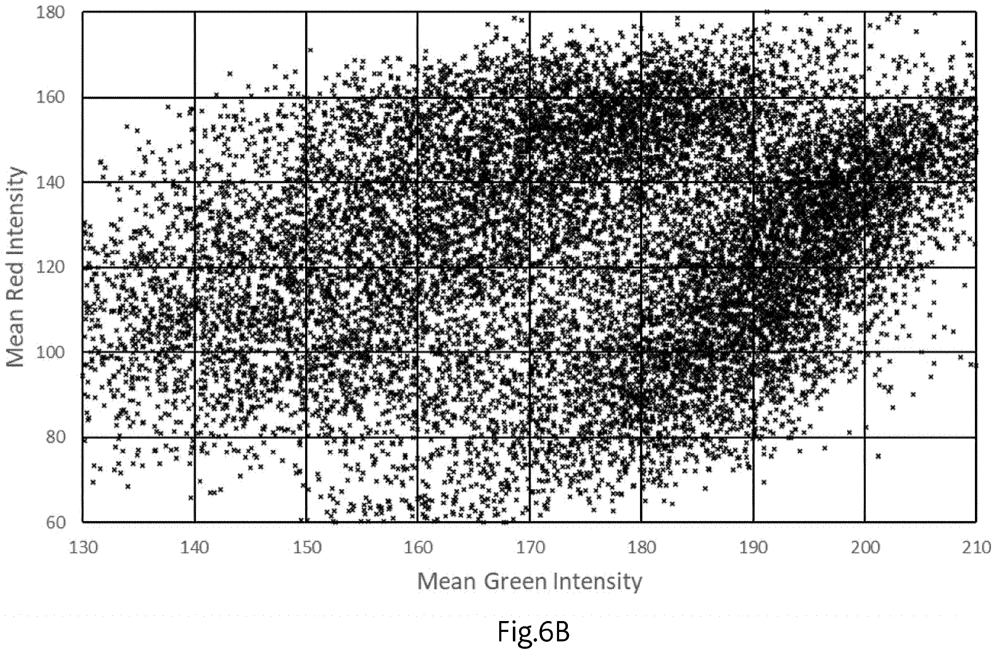

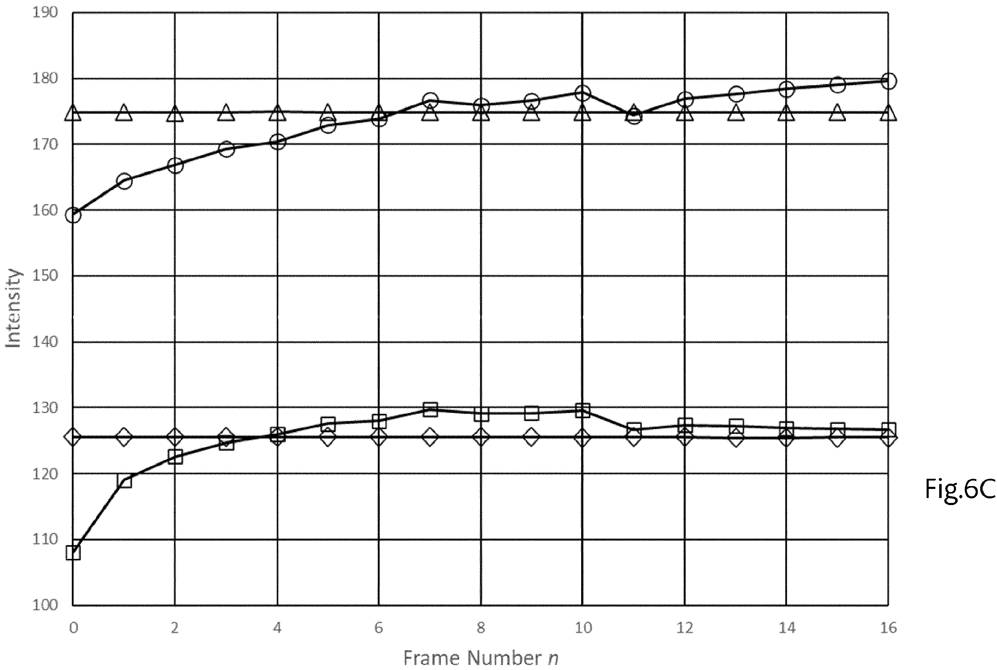

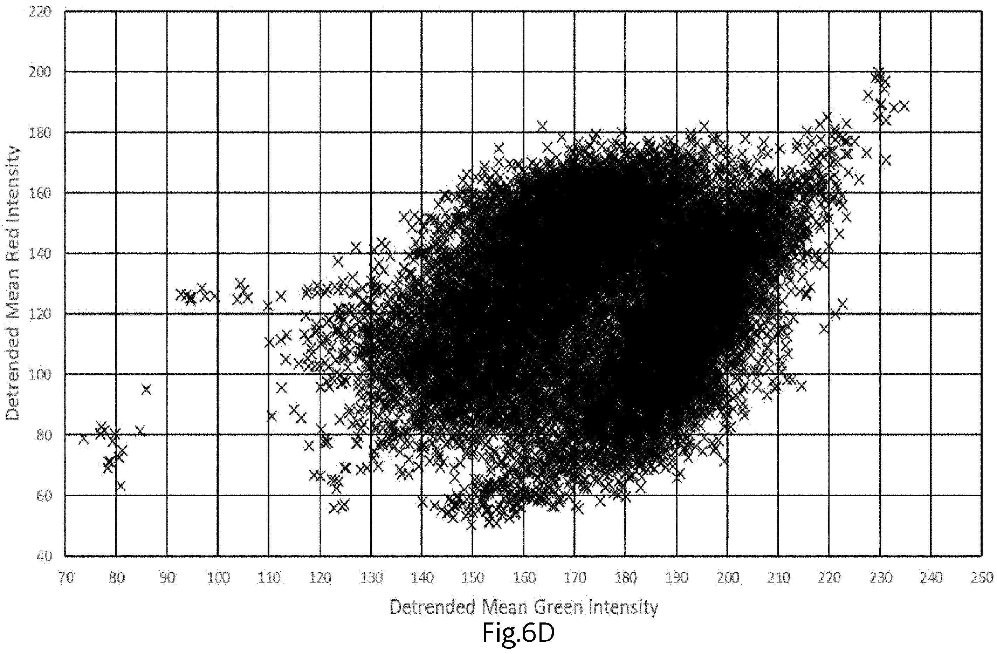

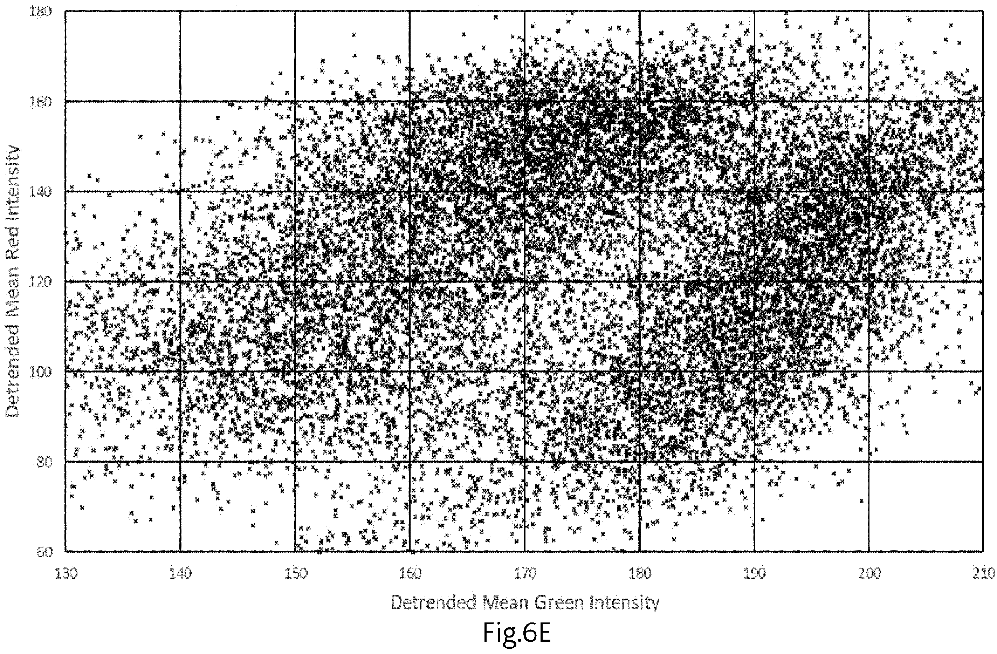

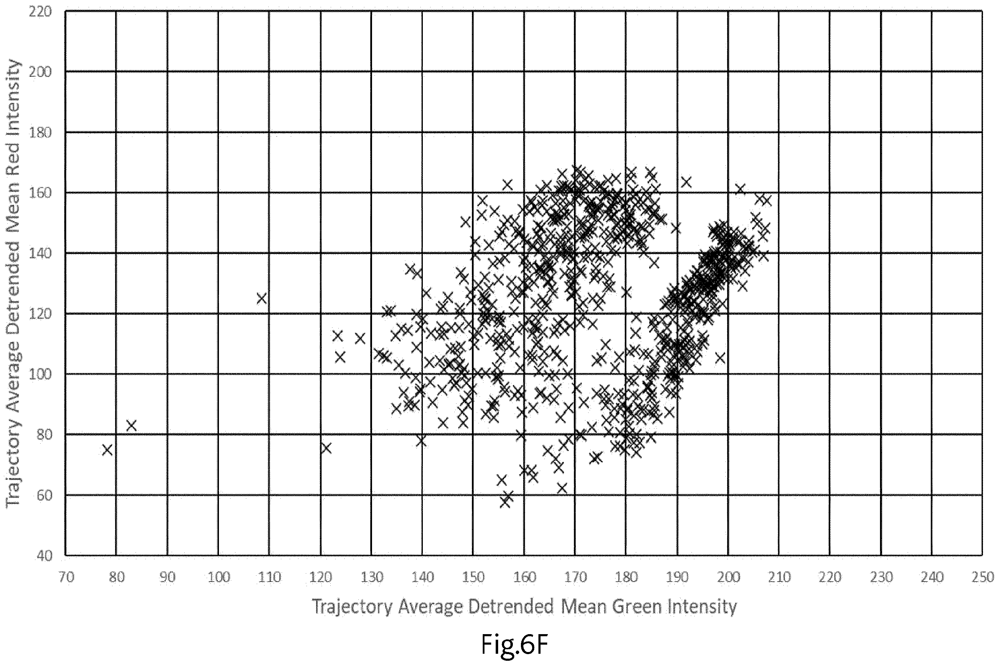

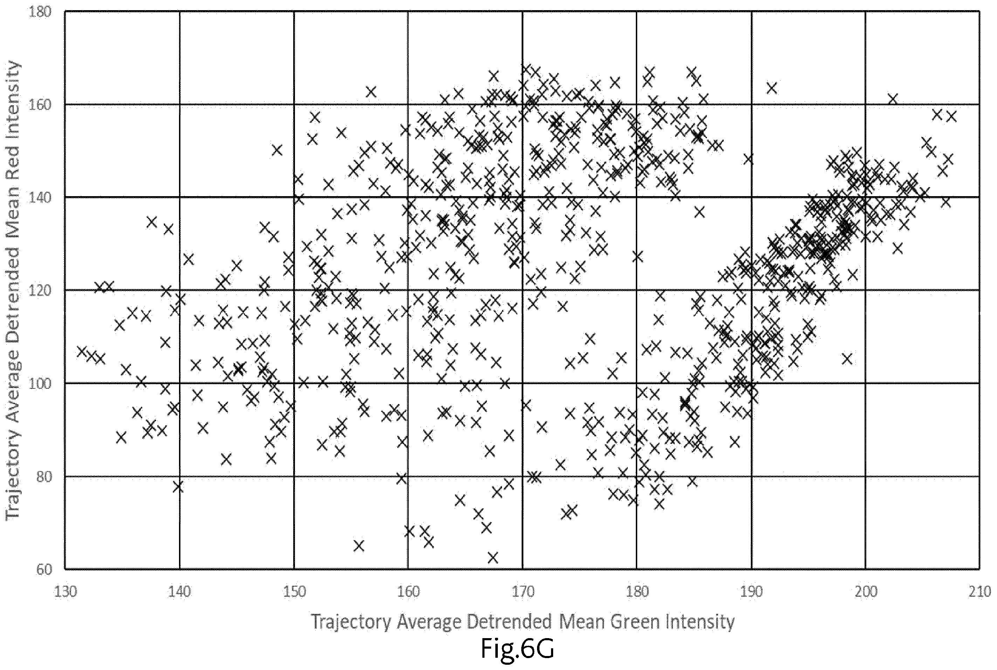

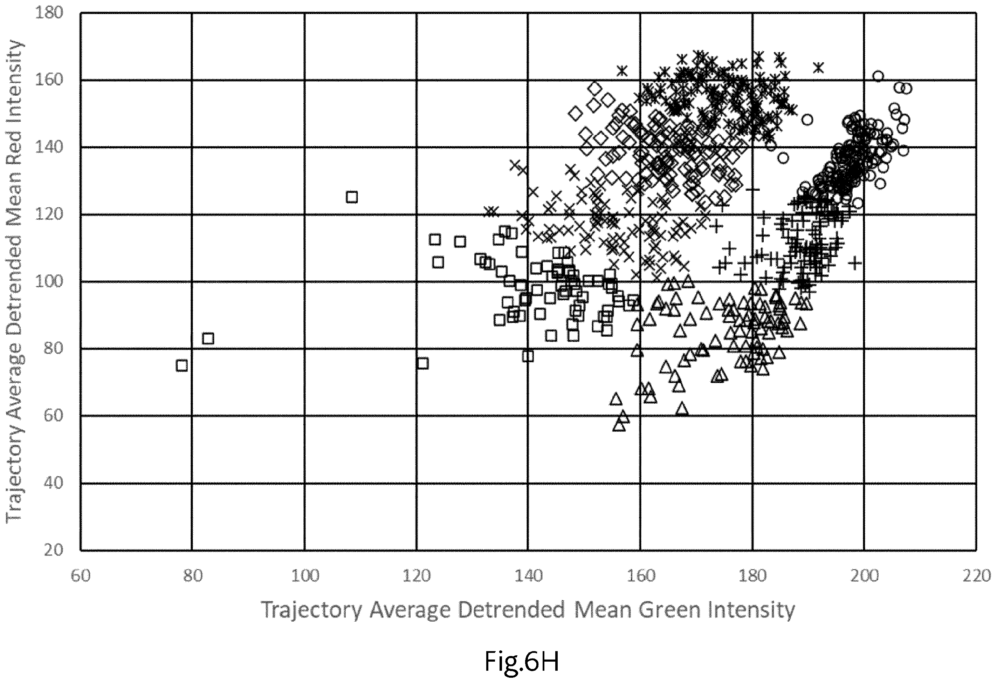

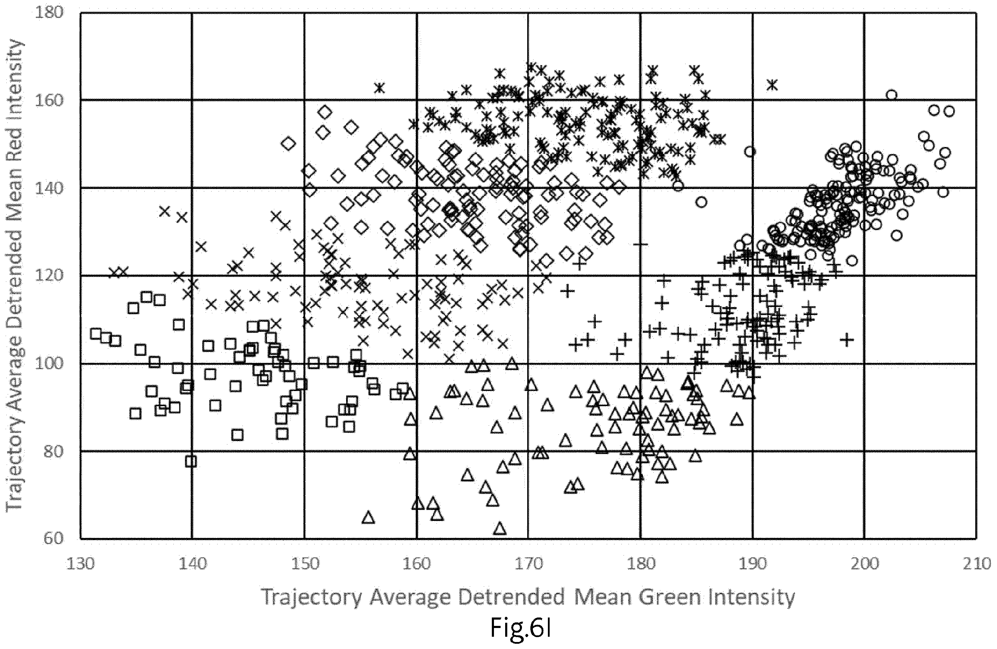

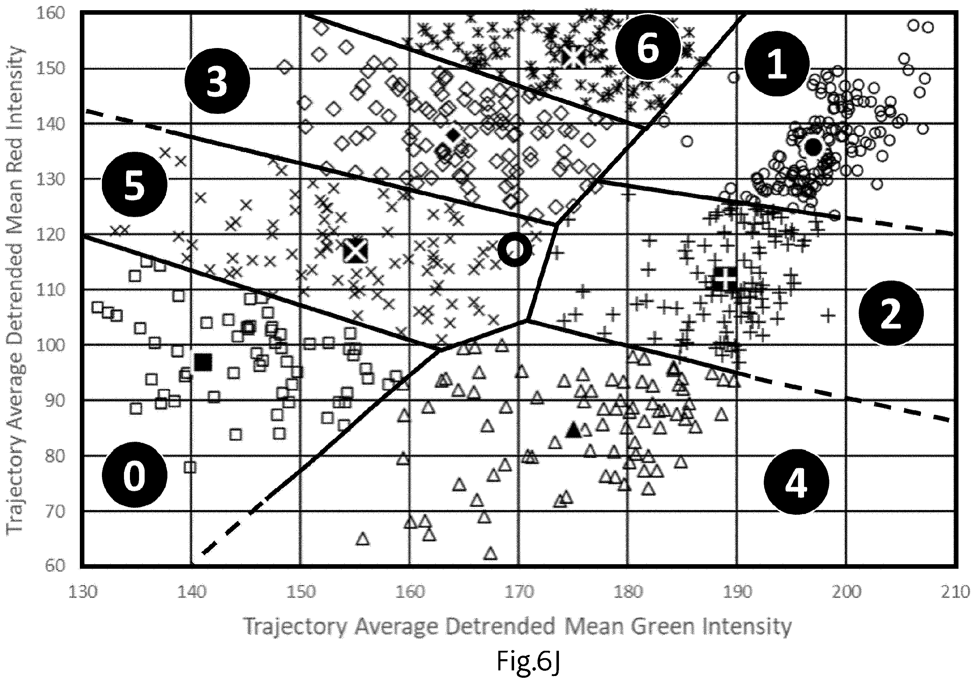

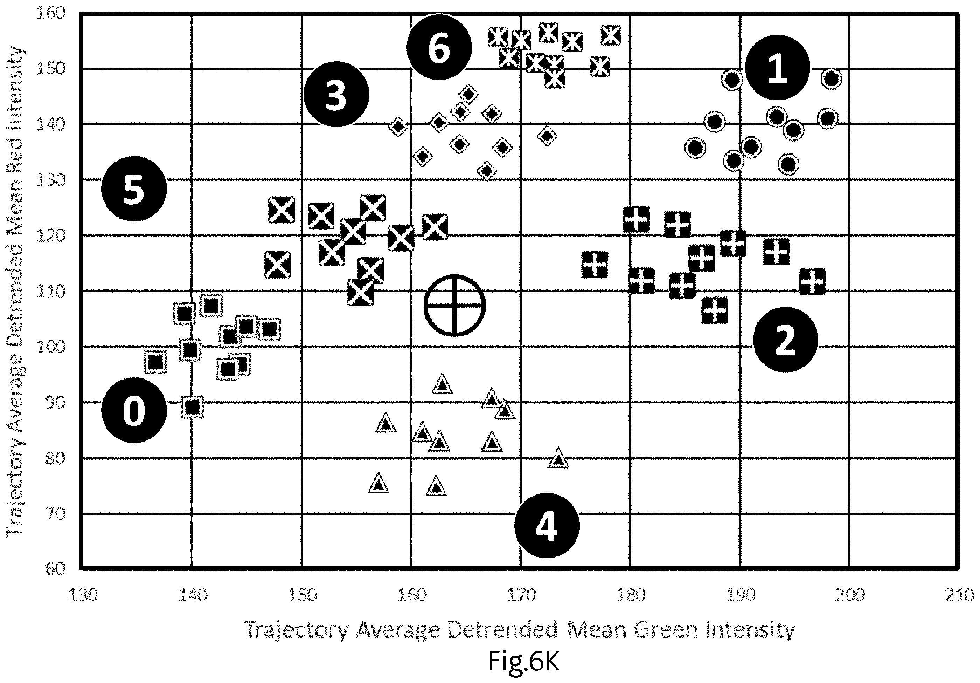

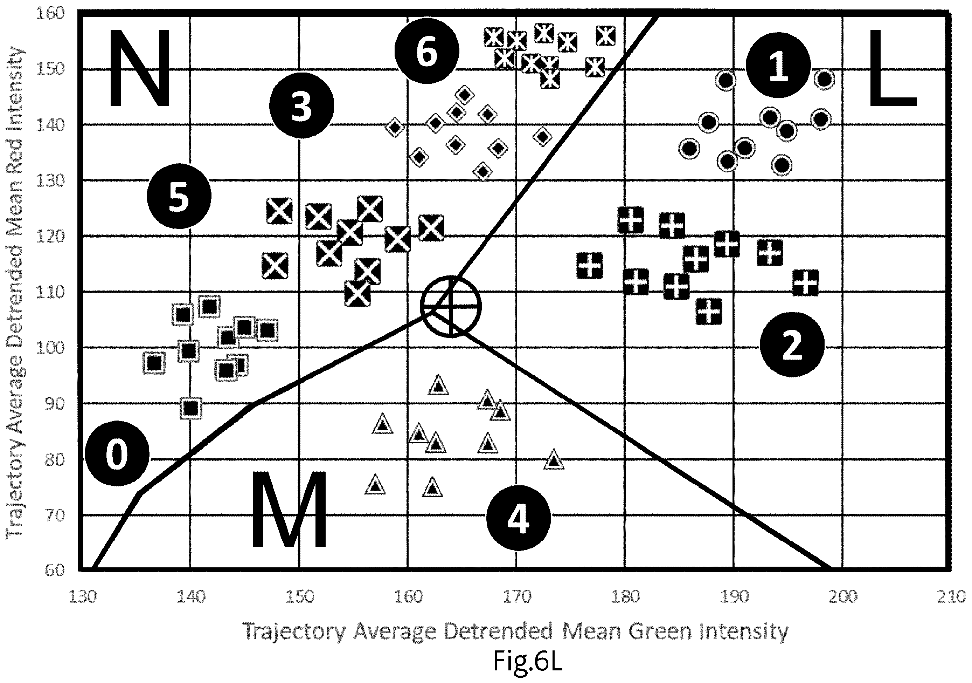

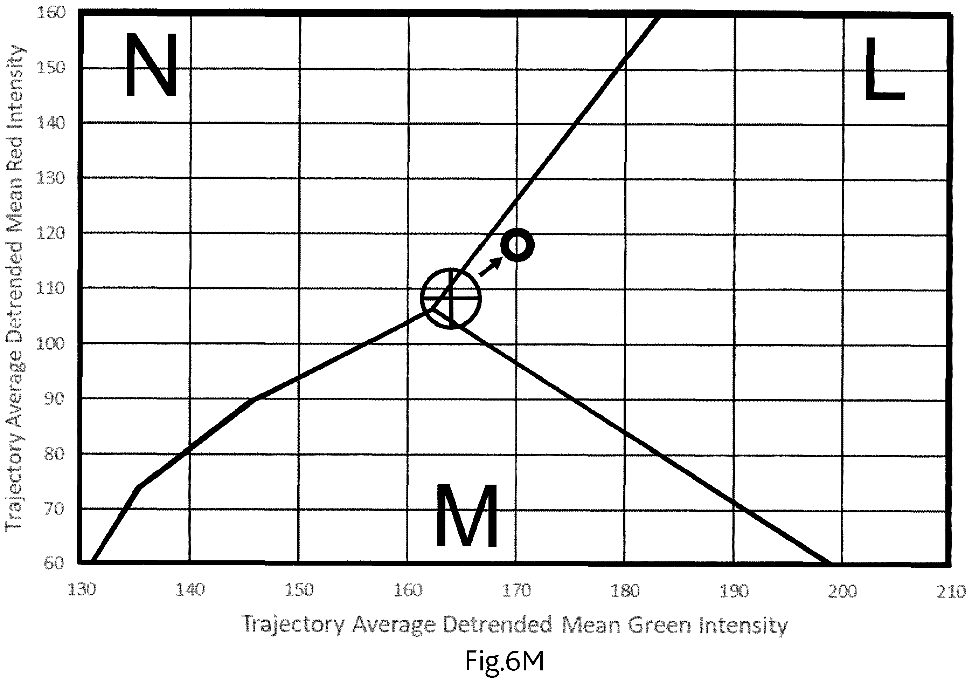

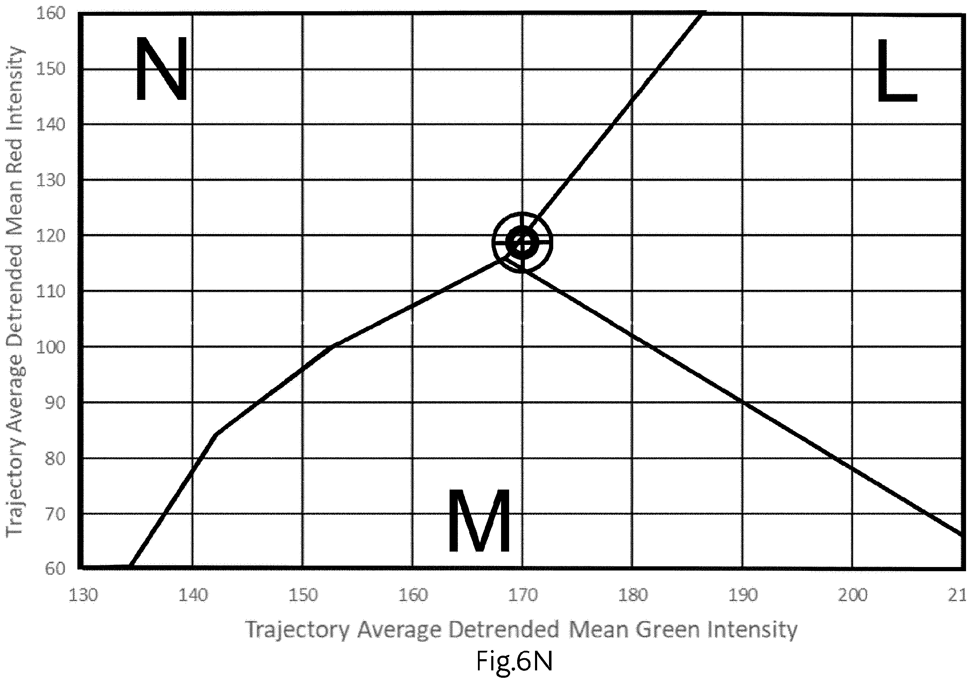

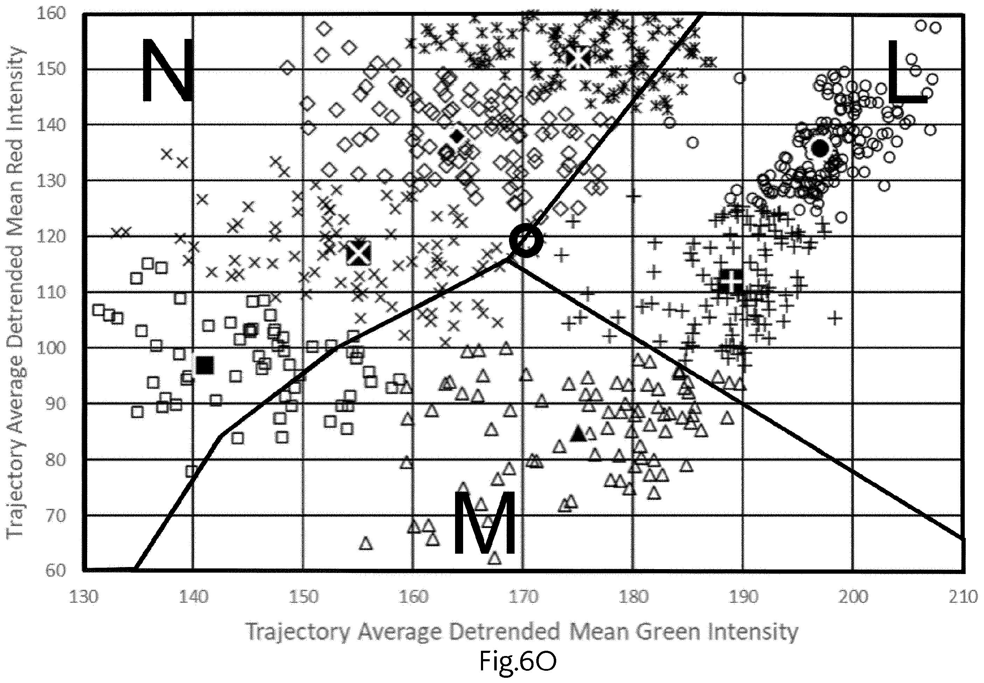

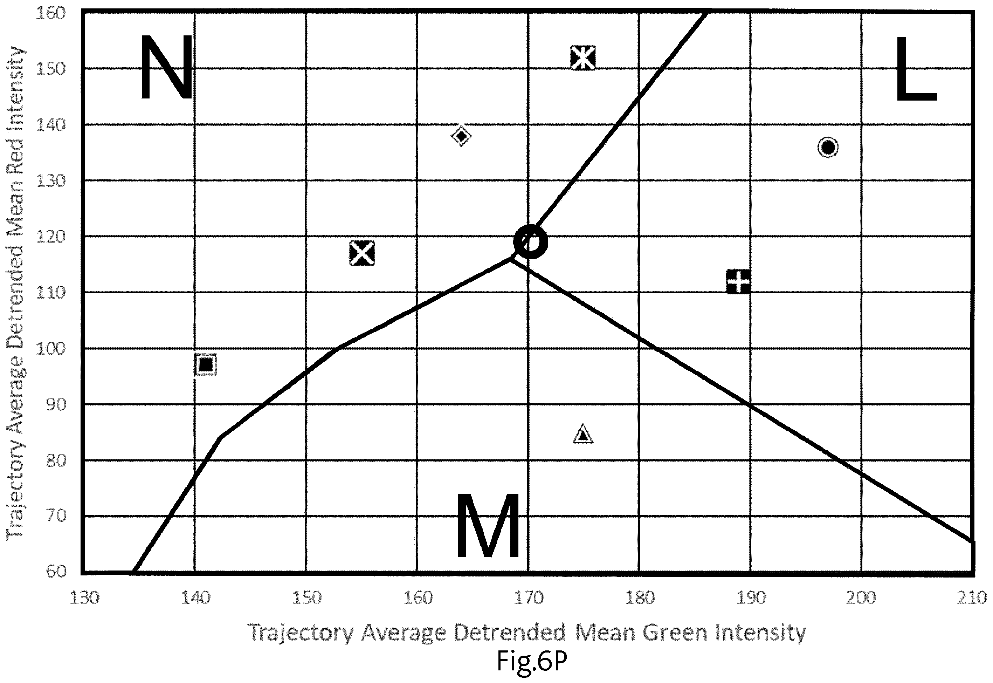

**Figure 6****:** Example measurement showing individual steps of an exemplary embodiment of analyzing the fluorescence image series. A) Scatter plot from an example measurement, showing mean green and red intensities of cells, for all cells in all frames. B) Detailed view of the scatter plot of the mean green and mean red intensities of the cells, for all cells in all frames. C) Multi-plot showing the original mean image intensities and the resulting detrended mean image intensities for the red and green channels. Legend: Mean Green (O); detrended Mean Green (Δ); Mean Red (□); detrended Mean Red (◊). D) Scatter plot of detrended mean green and detrended mean red intensities of cells, for all cells in all frames. E) Detailed view of the scatter plot of detrended mean green and detrended mean red intensities of the cells, for all cells in all frames. F) Scatter plot of the trajectory averages of the detrended mean green and detrended mean red intensities of the cells. G) Detailed view of the scatter plot of the trajectory averages of the detrended mean green and detrended mean red intensities of the cells. H) Result of k-means clustering of all COTs determined from detrended green and red mean intensities of cells. Legend: Cluster 0 (□), Cluster 1 (O), Cluster 2 (+), Cluster 3 (◊), Cluster 4 (Δ), Cluster 5 (X), Cluster 6 (*). I) Detailed view of the result of k-means clustering of all COTs of the detrended green and detrended red mean intensities of the cells. J) Results of k-means clustering of all COTs, with COCs and COAC, overlaid. Legend: COAC (∘); COC 0 (■); COC 1 (•); COC 2 (+); COC 3 (◆); COC 4 (▲); COC 5 (X); COC 6 (*); Cluster Region number (①). K) Factory pre-calibration with 10 different blood samples. Results of k-means clustering of all COTs, with COCs of all samples and the average of the COAC of all samples (⊕); COCs of the same cluster are marked with the same symbol, cluster numbers are also given. L) The boundary lines between the regions identifying Neutrophils (N), Lymphocytes (L) and Monocytes (M), which were determined empirically, are also given. M) Class Labelled Map used as a reference for assigning COC of k-means clusters to a class. The average COAC of the reference map is translated to match the COAC of the user map before assigning COCs to classes. Legend: Average COAC of Class Labelled Map (⊕); COAC of user map (○); Map translation direction to correct for offset (→). N) Offset corrected Class Labelled Map, with the average COAC of the reference map collocated with the COAC of the user map, used as a reference for assigning COC of k-means clusters to a class. O) Final class labelled map with k-means found clusters of COTs, with COCs and COAC overlayed, showing COTs and COCs. P) Plot from panel L, showing only the COCs.

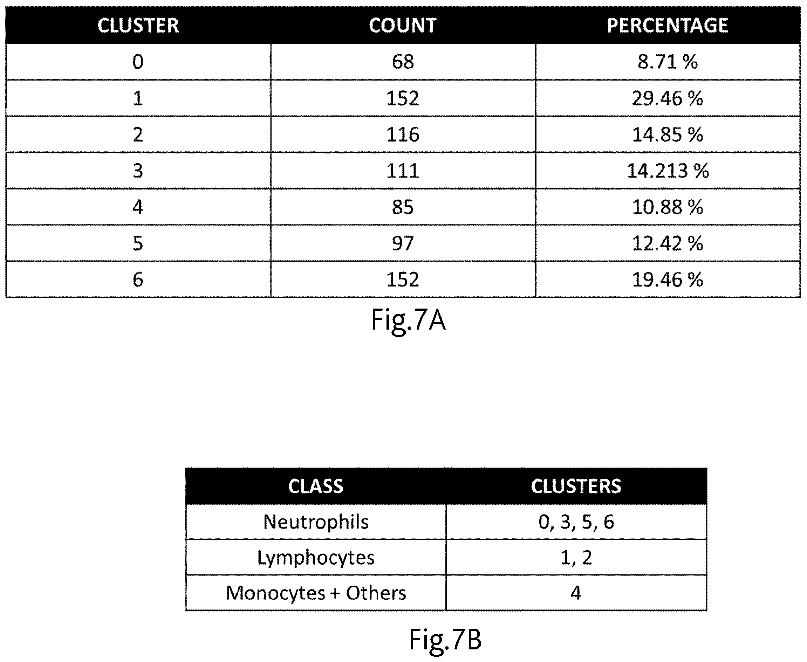

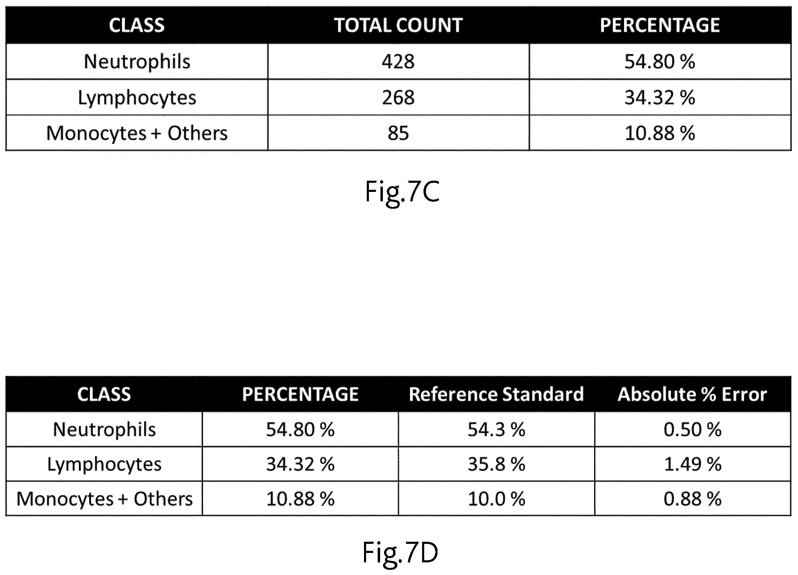

**Figure 7****:** Results from Example 1. A) Table showing the population (number) for each k-means cluster. The percentage of the total population is also shown. B) Table showing the class assignment of each cluster as determined from matching the COAC of all k-means clusters to the COAC of the class labelled map. C) Table showing the total number for each class and the final percentages of each class relative to the total population of all classes. D) Table showing the final percentages compared to the results of the laboratory reference standard. The absolute percentage error is also given.

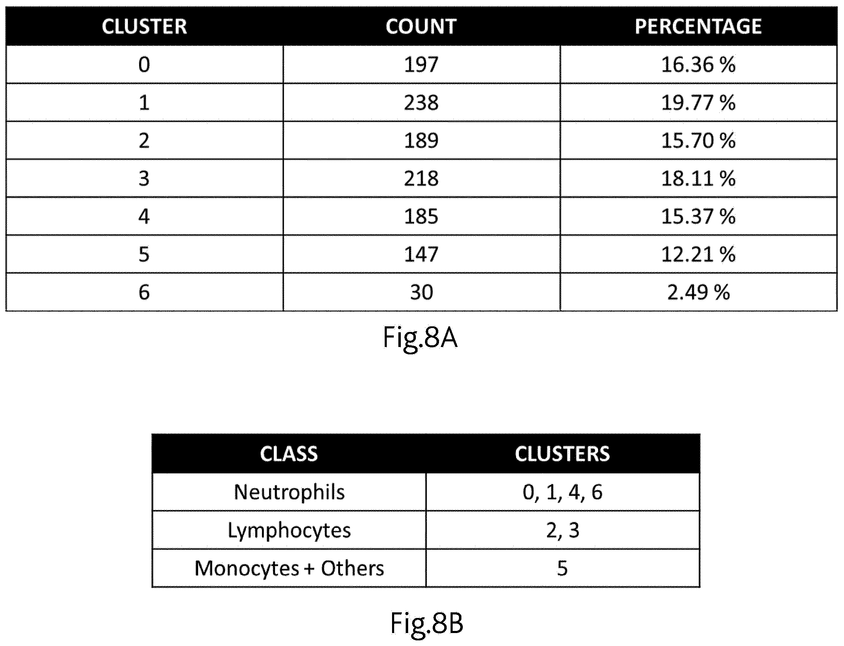

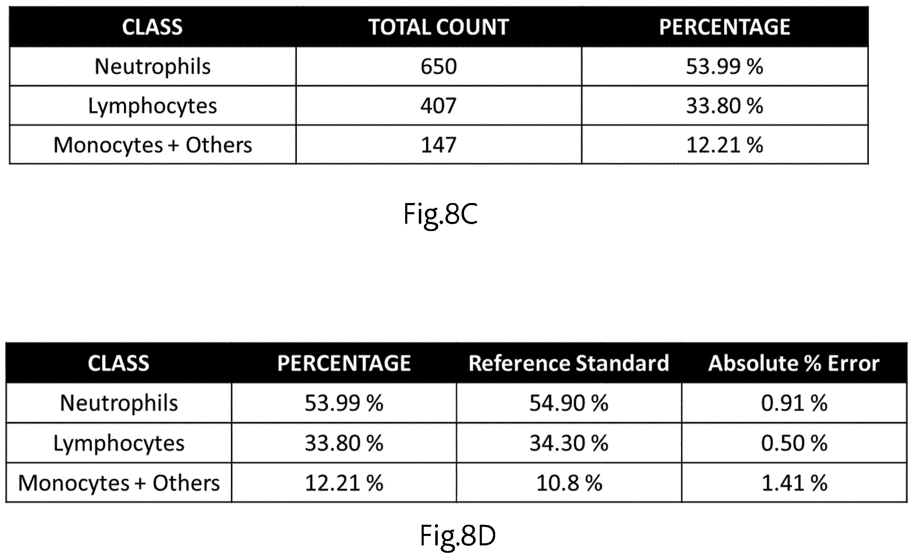

**Figure 8****:** Results from Example 2. A) Table showing the population (number) for each k-means cluster. The percentage of the total population is also shown. B) Table showing the class assignment of each cluster as determined from matching the COAC of all k-means clusters to the COAC of the class labelled map. C) Table showing the total number for each class and the final percentages of each class relative to the total population of all classes. D) Table showing the final percentages compared to the results of the laboratory reference standard. The absolute percentage error is also given.

In the following, preferred exemplary embodiments of the invention are described in more detail with reference to the figures, without limiting the invention thereto.

Blood samples taken from a patient contain different types of blood cells 1 that perform different functions (see Fig. 1A). Blood cells are typically described by a) the presence (or absence) of a nucleus 1a and b) the cytoplasm 1b. The many different types of WBCs 1c can be described by shape 1a and the relative size ratio of nucleus 1a to cytoplasm 1b.

Three cell types that are critical to the proper functioning of the human immune system are polymorphonuclear neutrophil granulocytes (neutrophils), lymphocytes, and monocytes. Neutrophil granulocytes are generally described as having a multilobed nucleus and a large portion of the cell is filled with cytoplasm. In contrast, lymphocytes are generally described as having a round nucleus and a very small amount of cytoplasm. Monocytes are typically described with a single lobed nucleus. In contrast, red blood cells (RBCs) 1d do not have a nucleus and therefore have a very different shape from all other WBC cells described.

In an example measurement, a small volume of blood (e.g., 10 µL), preferably diluted by a factor D, is introduced via an inlet port 2c into a device 2 (shown in Fig. 1B) comprising a bottom plate 2b with an optically transparent window, and a non-reflecting cover plate 2a, which are sandwiched together with a channel-shaped recess under the interposition of a spacer 2f (with a thickness of, for example, 100 µm). The sample is introduced into the sealed measuring chamber 2d via an inlet opening provided in the cover plate 2a, with the air escaping through the vent opening 2e located in the cover plate 2a.

The dye, preferably AO, is added to a blood sample outside the measuring chamber, e.g., at a concentration of between 0.1 µg/mL and l µg/mL in dissolved form. Immediately after mixing, 10 µL of the blood sample is introduced into the measuring chamber via inlet 2c. It has been found that a concentration of the dye in the blood from 0.1 µg/mL to l µg/mL is particularly well suited, since the WBCs take up the dye and remain viable, in the sense that the WBCs are still able to crawl along the surface of the channel-facing surface of the optically transparent bottom plate 2b.

Fig. 1C shows an optimal arrangement of cells in the measurement chamber 2d, with a layer of cells forming on the channel-side surface of the optically transparent bottom plate 2b. The layer of WBC cells, preferably a monolayer of cells, is preferably achieved by diluting the blood sample before placing it in the measurement chamber 2d. The blood sample can, e.g., be diluted with a) autologous plasma (e.g., from an aliquot of the same blood sample) or b) plasma balanced salt solution (e.g., Hank's Balanced Salt Solution (HBSS)). After filling the measurement chamber 2d, the cells settle by sedimentation (typically within two minutes for a 100 µm high measurement chamber) on the upper surface of the optically transparent plate 2b. In this configuration, luminescence images of the WBCs can be acquired by a combination of camera, mounted below the bottom optically transparent plate 2b (see Fig. 2), suitable narrow-band illumination at the excitation maximum wavelength of the dye AO and the use of a long-pass filter, with a cutoff at a lower wavelength than the emission maximum wavelength of AO, placed between the measurement chamber 2d and the camera. Red blood cells 1d are not observed in fluorescence images because AO in 1d produces fluorescence with a much lower fluorescence quantum yield compared to AO species formed in 1a and 1b. Hence red blood cells 1d remain very dark relative to the bright emission from white blood cells 1c.

Fig. 2 shows cells 1c that have attached to the upper surface of the optically transparent plate 2b and undergone a change to their shape. Unlike erythrocytes 1d, WBCs 1c can attach to surfaces and begin to crawl along the surface in a random walk. The cytoplasm 1b of WBCs can create prominences, which help cells actively crawl along surfaces and are commonly called pseudopods. Cells that can form larger pseudopods can move in larger distance increments while crawling and therefore can potentially crawl faster than cells with smaller pseudopods. There are several mechanistic models proposed for the observed amoeboid cell motion of WBCs, including a) sol-gel conversions, b) actin-driven mobility and c) bleb-driven mobility. All these proposed methods require an initial WBC attachment to a surface before motion can begin. For this purpose, it is preferred if at least one window region of the bottom panel 2b is made of an optically clear, transparent material to which WBC cells can readily attach. Cell attachment can be enhanced by selecting appropriate materials and/or surface treatments to improve attachment. Suitable materials and surface treatments are known e.g. from Shen and Horbett (J. Biomedical Material Research 57.3 (2001): 336-345), Ramsey et al. (In Vitro 20.10 (1984): 802-808), and Curtis et al. (The Journal of Cell Biology 97.5 (1983): 1500-1506). Plastics, in particular polystyrene, are particularly well suited in this context.

In the exemplary embodiment described here, the material choice for 2b allows the luminescence excitation light, emitted from a plurality of narrow-band light emitters 4, filtered by 10-nm narrowband pass filters 5, and centered around 470 nm, to uniformly illuminate 2d and reach the WBCs. Furthermore, it is preferred that the material choice for 2b is transparent and optically clear, in the visible and near-infrared wavelength range, in order to allow luminescence light emitted by 1c to be collected and imaged by a color sensitive camera. In other embodiments, the test surface may also be opaque; e.g., when the cells are imaged from above.

In the preferred embodiment described here, AO is used as a luminescent dye. When AO is taken up by 1a or 1b, the fluorescence quantum yield of AO increases dramatically (especially AO species that form in 1a). The amount of dye taken up by a cell is proportional to the concentration of AO in the blood sample. Initially, dye uptake increases with time until, after a few minutes, a kind of equilibrium is reached between the concentration in the cell and the concentration in the plasma outside the cell. Two distinct fluorescent species of AO form in the different environments present in the two major components of the cell, nucleus 1a and cytoplasm 1b. The green fluorescent species of AO (excitation maximum: 502 nm; emission maximum: 525 nm) forms in the nucleus 1a of WBCs. The intercalation of AO in its monomeric form into the DNA strands present in nucleus 1a significantly increases the fluorescence quantum yield of the species and allows it to fluoresce with much higher intensity compared to its dimer or aggregate form in cytoplasm 1b. On the other hand, the red fluorescent AO species (excitation maximum: 460 nm; emission maximum: 650 nm) formed in cytoplasm 1b shows an increase in fluorescence intensity, associated with a pronounced red shift in fluorescence (and a blue shift in light absorption) due to the formation of protonated AO, combined with a bathochromic red shift in fluorescence in the acidic environment found in sub-components of cytoplasm 1b, such as lysosomes and granules. In addition, the interaction of AO with RNA in cytoplasm 1b also causes red fluorescence. This differential fluorescence staining by AO provides a highly suitable basis for the identification of different WBC types. See, e.g., the publications Jackson (Blood 17.5 (1961): 643-649), and Adams and Kamentsky (Acta Cytologica 15.3 (1971): 289-291).

In the preferred embodiment shown in Fig. 2, fluorescence light emitted by AO in cells 1 is collected by a camera lens 6 (e.g., a macro lens) mounted on a color camera 8 before being filtered by a long-pass filter 7 with a cutoff wavelength ending at 500 nm and focused onto the imaging plane of a single-chip color camera sensor 8b. The single-chip color camera sensor comprises of a monochrome image sensor with additional pixel-by-pixel applied transparent red, green and blue Bayer color filters 8a mounted on the surface of the camera sensor 8b, whereby the surface of 8b is located at the imaging plane of the camera lens 6. The combination of monochrome sensor 8b and standard color Bayer RGB filter (mounted directly on sensor 8b) typically forms the basis of a color RGB camera.

The image acquisition procedure typically begins with setting the exposure time of the camera 8c to a duration such that a) fluorescence from AO species in 1a and 1b can be detected and b) fluorescence from AO species in 1a and 1b does not reach the saturation light level of the sensor. In practice the exposure times preferably range between 1 millisecond and 50 seconds, especially between 500 milliseconds and 10 seconds. Next, the interval duration 8c between trigger events for both camera and light sources is set to range preferably between 1 second and 1200 seconds, especially between 60 seconds and 300 seconds. The values chosen for the interval duration and exposure time is chosen such that the exposure duration does not exceed the interval time. The acquisition control repetition number 8c sets the number of images N acquired for a sequence. The total duration of the sequence acquisition is thus determined by the interval duration and N. The value for N is chosen such that the total time of the sequence acquisition ranges preferably between 300 seconds and 3600 seconds; however, also shorter (e.g., 30 seconds) or longer time periods are possible. The acquisition settings 8c, are used by the exposure controller and interval timer 8d, to send timely trigger signals to the illumination lights 4 and trigger signals to the camera 8, to ensure the cells 1 in 2d are illuminated during the camera exposure. The color camera 8 is capable of directly detecting, or via interpolation (e.g., RGB Bayer filter-based cameras), multiple color components of the incoming light per sensor pixel (this may, or may not be, in combination with camera steered dynamic changes to the filter 8a). For this purpose, the camera 8 may acquire internally a single image or multiple images, for each camera trigger event received from 8d, that are then processed by 8e to deliver one multi-channel color image per acquisition event. The number of trigger repetitions N sent by 8d results in N individual multi-channel color images being stored in 8f. A typical three-channel color image outputted by 8e is a color RGB image obtained by placing a RGB Bayer filter at 8a. The RGB values of each pixel of the resultant image, outputted by 8e, are calculated from the values of the three color components of light detected by the Bayer-filtered color pixels of 8b, through a combination of pixel interpolation and deconvolution of the various intensity values of the red, green and blue sensor pixels, to create the resultant values for each color channel, per pixel of the output image. The color camera 8, in combination with 8e, is capable of outputting images with sufficient color channels to accurately describe and distinguish the apparent colors of fluorescence from the AO species found in 1a and 1b. All N individual multi-channel color images are stored temporarily in 8f, until further processed by software 8g to measure color channel intensities for each WBC, classify each WBC and deliver the final differential cell count results.

As mentioned above, the fluorescence of AO in nucleus 1a has a maximum at 525 nm (green) and the fluorescence of AO in cytoplasm 1b has a maximum at 650 nm (red). Since the cell nuclei and the cytoplasm of the different cell types (leukocytes, monocytes, etc.) have different proportions and quantity ratios, the fluorescence intensities of the pixel areas imaging the respective cell nucleus 1a and the fluorescence intensities of the pixel areas imaging the respective cytoplasm 1b of the different cell types have a different ratio of the intensities of 525 nm and 650 nm light. The intensity of fluorescence emission measured from nucleus 1a and cytoplasm 1b is essentially determined by a) the volume of nucleus 1a and cytoplasm 1b, respectively, b) the amount of dye absorbed by each component, c) the angle of incidence, wavelength, and intensity of excitation light, and d) the angle of observation and wavelength of measured fluorescence light, with the parameters listed in c) and d) held constant. In addition, variations in the acidic environment and/or RNA concentration in cytoplasm 1b also lead to variations in the fluorescence intensities of the light emitted from cytoplasm 1b.

These subtle differences in relative fluorescence emission allow differential classification of WBCs, preferably into 3 classes or cell types: neutrophils, lymphocytes, and monocytes. This is referred to as 3-part differential cell counting (DCC). Other, less common WBC types, such as eosinophils and basophils, excluded in a 3-part DCC and grouped as "other" with monocytes, are more similar to neutrophils (Fig. 3A) than lymphocytes (Fig. 3C) in terms of their cell structure.

The two different structural WBC groupings: (a) neutrophils, monocytes and others, and (b) lymphocytes, are shown from a bottom and a side view perspective in Fig. 3B and Figure 3D, respectively. WBCs, settled via sedimentation onto the optically transparent substrate surface 2b, which is preferably of a material, or surface composition, that favors cell attachment, remain in a surface adherent state while crawling on the surface of 2b.

The WBCs shown in Fig. 3B and Fig. 3D are cells that are adhered to a surface and are either a) crawling or b) remaining effectively stationary. Viable cells can appear stationary even though they can, and do regularly, probe their surroundings by extending and retracting pseudopods successively in various directions. As shown diagrammatically in Fig. 3B, a typical characteristic of neutrophils is the ability to do large extensions of the cell membrane to form a long pseudopod. This enables neutrophils to do a larger step distance when crawling relative to the maximum step distance lymphocytes can achieve with their proportionately smaller amount of cytoplasm (see Fig. 3D). The biological role of WBCs is not to remain circulating in the blood, but rather to leave the vascular system at the site of an infection. Hence, all WBCs are capable of crawling whence they leave the circulatory system and enter the extra-cellular matrix of surrounding tissue.

Every movement of a cell, through for instance pseudopod extension and contraction - whether accompanied by forward crawling or not, will lead to internal changes over time in the relative positions of cell components 1a and 1b, and their respective sub-components therein. From the fixed perspective of the camera 8 with respect to the measurement chamber 2d, these changes are observed as variations to the red color channel and green color channel intensity values, per pixel within a cell boundary in an image, when examined over all N successively acquired images which are part of the image sequence.

Therefore, with all other factors kept constant as described previously, the quantity and relative proportion of cell components, and their respective sub-components therein, will determine the average/mean intensity and relative proportion of red and green light measured by the camera 8 for each cell 1 in the measurement chamber 2d. As shown by the differences between the bottom and side view perspectives in Fig. 3B and Fig. 3D, the perspective from which a cell is viewed changes the observed relative position, and observed arrangement, of cell components 1a and 1b, and their respective sub-components therein. Therefore, it is the three-dimensional structure of a cell, when viewed through the camera lens' 6 two-dimensional projection of the scenery, from the current fixed camera's perspective in relation to the cell, that causes the observed changes when a cell is either a) crawling or b) extending and retracting pseudopods while remaining effectively stationary.

Over time cell movements cause the current perspective for each frame of the acquired image sequence to change. These changes over time of the camera-to-cell perspective, lead to variations in the intensity of red and green fluorescence light detected by the camera from the cell components 1a and 1b, and their respective sub-components therein, for each cell in 2d. Therefore, when a whole image sequence is acquired over time, some, or all the possible camera-to-cell perspectives will be observed and imaged by the camera.

As described previously, in the described embodiment, a blood sample in the measurement chamber 2d contains three main types of WBCs, each of which have a characteristic cell composition of, and proportion of, cell components 1a and 1b, and their respective sub-components therein. Hence in terms of fluorescence imaging, each cell type will have a characteristic red color range and characteristic green color range of possible intensity values, that can be observed, when a cell is imaged from all possible camera-to-cell perspectives.

As a result of the different arrangement and quantity of the cell components and sub-components, the green and red intensities for each cell type typically vary only within a limited range of values. These color range boundaries of each cell type have a characteristic envelope in red-green color space that form the basis of the 3-part differential cell count cell classification method described in this embodiment. If a measured cell's color ranges are found to lie within the range of color values that describe the characteristic color range envelop of a given cell type, then this cell can be classified as belonging to that cell type.

Using software to track each cell in measurement chamber 2d as it moves in the xy-plane over time (in the acquired image sequence), allows the red and green color range boundaries of each individual cell to be measured. Tracking the spatial movements of cells allows to measure the same cell from multiple camera-to-cell perspectives, and hence obtain part of or the whole of the cell's color range boundaries. Example images illustrating the dynamic fluctuations of a cell's surface during crawling and/or during changes in crawling direction are given in Fritz-Laylin et al. (Elife 6 (2017): e26990) and show the formation of complex pseudopods in three dimensions.

Variations of red and green fluorescence intensity of a cell, measured using cell tracking over all images of the acquired sequence, can be plotted in the form of a scatter plot in the red-green color space, as shown in Fig. 4. For each acquired image in a sequence, each cell's color parameters can be measured and plotted as a coordinate in the red-green color space (each coordinate is shown with a • symbol). Thus, changes from image to image of each individual cell, over the captured image sequence, describe the trajectory of the cell in color space. This is in addition to the spatial trajectory of the cells crawling in xy-space. There are multiple color parameters that can be measured and plotted such as: mean intensity of the cell, total integrated intensity of a cell and variance of the cell intensity. The mean green and mean red intensities of cells are plotted in Fig. 4 and are used in all further examples and discussions presented here.

Also shown in Fig. 4 are the positions of the first measurement point (shown with a ■ symbol) and the Centroid-of-Trajectory (COT) of each trajectory - calculated by determining the centroid coordinate for all data points in a given trajectory. Determining the coordinates of the centroid can be achieved by for instance a) by minimizing the squared distances of all trajectory points to the centroid or b) calculating the trajectory averages of mean green intensity and mean red intensity. This COT coordinate is plotted in Fig. 4, for each trajectory, with a triangular symbol (A symbol).

A preferred embodiment of the computational means for data evaluation and displaying results (8g in Fig. 2) is shown schematically in Fig. 5A. This involves the conversion of the image information from the acquired fluorescence image sequence into a classification result of a 3-part DCC. According to this embodiment, data evaluation is performed in three parts. In Figs. 5B, 5C and 5D, these three parts are shown schematically in more detail.

Part I, shown schematically in Fig. 5B, includes the preprocessing steps required to convert the image information into an acceptable format for Part II.

Part II, shown schematically in Fig. 5C, includes the processing steps used to track the changes in mean red and mean green intensities for a cell over its trajectory in xy-space.

Part III, shown schematically in Fig. 5D, comprises the processing steps by which the red-green color space trajectories of individual cells are compared with the red-green color space trajectories of all other measured cells and classified. This allows each individual cell to be assigned to a class, which in turn allows a final classification of all cells in the form of a 3-part DCC.

All steps of each part of this preferred exemplary algorithm are described below, with reference to two exemplary processed data sets, described below in Examples 1 and 2, where appropriate.

In Part I of the algorithm (see Fig. 5B), the initial process of capturing images with camera 8, processing and storing N multi-channel color images 8f has already been described and are shown in Fig. 2. Although various color images could be used, here in this example, typical RGB color images, acquired with a Bayer color filtered single chip sensor camera, are shown. As shown in Fig. 5B, the N captured images are converted into a useable image format (for instance conversion from camera RAW format to TIFF format) 12a to form the batch of RGB images 12b that are to be processed. A copy of each image is converted to grayscale 12c using a grayscale conversion based on the RGB weights for each pixel. This grayscale image is then segmented using a Renyi entropy segmentation method 12d, e.g., as described in Kapur et al. (Computer Vision, Graphics, and Image Processing 29.3 (1985): 273-285), which produces a binary image for each image in the stack of N images showing the exact location of pixels belonging to cells in the image with white pixels (on a black background). This stack is processed in 12f to produce a sequence of binarized images 12g. This sequence of binarized images is used to track each cell over N images to determine the trajectory m for each cell. The segmentation algorithm step 12d also generates a list of M regions-of-interest (ROI) 12e that describes the spatial outline of each cell detected by the segmentation method. This list of ROIs is later used to delineate the boundaries of a cell in order to perform an intensity determination on each cell individually.

In Part II of the algorithm (Fig. 5C), a trajectory list of M entries is generated, with a list of red and green channel mean intensity measurements generated for each trajectory, with one entry for each of the N images in the acquired image stack 12b. The binary image sequence 12g is passed to a particle tracking algorithm 12s that can associate each segmented cell (in the binary image) in each image of the sequence with a trajectory describing the movement of the cell over the entire binary image sequence, identified by the unique ROI entry for each cell 12e. A suitable particle tracking algorithm is, e.g., described in Sbalzarini and Koumoutsakos (Journal of Structural Biology 151.2 (2005): 182-195). At the same time, a copy of the original image stack 12b is passed to 12h, along with an ROI list entry m. In Part II of the algorithm, the following processing steps of intensity measurement and tracking are repeated for each entry m until all M ROIs are processed.

The first processing step of the original image stack 12b is to place the ROI m as a mask on the original image to process each ROI m individually. Then, each color channel of an image of the stack 12i is divided into individual red and green channels 12j and 12k, respectively. The blue channel is ignored. The red and green channels are processed in the same way, but separately. The image stack (12o and 12l) is processed into one image sequence for each channel (12p and 12m), which is followed by a calculation of the mean intensity values of the ROI m of both color channels (12q and 12n). The mean intensity values over N images are combined 12r with the cell tracking values 12s to produce a list 12t of N entries, for each color channel, for the ROI m. The list is then processed for each color channel (12p and 12m). This process is now repeated for all M entries in the ROI list 12e, and the resulting lists of combined red and green mean intensity values are passed to Part III of the algorithm for classification.

In Part III of the algorithm (shown in Fig. 5D), the individual trajectories are classified to generate data in the form of a total count and a percentage of the total count for each cell type class. The two-color channel input lists 12u and 12x for all cells across all frames are plotted in the form of a scatterplot for one example in Fig. 6A, with no trajectory information given (a more detailed view of the plot is shown in Fig. 6B). The first processing step in Part III is to detrend the data for each channel to eliminate gradual collective intensity changes that affect all cells, equally and collectively. This may include initial increases in fluorescence intensity due to the initial phase of dye uptake by the cells and/or collective intensity changes over time due to bleaching of the AO dye by the excitation source 4. This is achieved by first determining the average mean intensity of all cells for each image n, for all N images - for each channel (12v and 12y). The average mean intensity is then used (12w and 12z) to determine the correction factor per frame n, for all N frames, which is applied to the list entries of 12u and 12x, separately (to both the red and green channel lists). This detrending process is shown in the multi-plot of Fig. 6C, and the resulting scatterplot of the detrended data in 12aa and 12ad is shown in Fig. 6D (a more detailed view is shown in Fig. 6E).

Next, to calculate the Centroid-of-Trajectory (COT) for each trajectory, the trajectory average of the detrended mean red 12ab and detrended mean green 12ae intensities are calculated from lists 12aa and 12ad to produce lists 12ac and 12af, respectively.

These lists for each color channel are then combined 12ag to create coordinates in red-green color space for the COTs for all trajectories. These coordinates can optionally be plotted for illustration purposes, in the form of a scatterplot as shown in Fig. 6F (a more detailed view is shown in Fig. 6G).

Trajectories can now, optionally, be assigned to a cell type class by comparing the coordinate position of each COT to the specific class regions of a pre-calibrated class labelled color-space map. The number of COTs located within each class region's boundaries is used to determine the population of each class, and hence output the results of the 3-part differential cell count.

A further option is to use an adaptive method to ensure more accurate classification. The first step of the adaptive method is to apply a k-means clustering algorithm to the COT coordinates 12ah using an empirically determined k value. The result of k-means clustering 12ak can optionally be plotted for illustration purposes, as shown in Fig. 6H using k = 7 and shown in more detail in Fig. 6I. At this stage, a quality test 12aj can optionally be performed by checking the correlation of the individual trajectories coordinate list 12ai to the COTs clusters found with k-means clustering 12ak. To evaluate numerically how well each trajectory maps into positions in color-space within the boundaries of each k-means found cluster of COTs, the number of individual trajectories coordinates 12ai, which follow the criterium that all coordinates of the individual trajectory lie within the boundaries of the k-means found clusters of COTs. The quality parameter is then calculated as the ratio of the number of trajectories that fulfill the criteria to the total number of trajectories in the quality evaluation. A binary output of either passed or failed 12al is determined by assessing if the quality parameter calculated ratio is larger, or smaller, than an empirically pre-determined minimum acceptable threshold ratio 12aj, respectively.

The next step is the classification of each cluster, found by k-means clustering, by assigning each cluster to a WBC type class. The first step towards classification is to determine, by calculation, the Centroid-of-Cluster (COC) of each k-means found cluster 12am. Next, the resulting COCs are then used to determine the Centroid-of-All-Clusters (COAC) 12an. The results of these two steps are shown in Fig. 6J.

One option to achieve classification is to perform a factory side calibration. The calibration process is based on acquiring and analyzing blood samples from a multitude of people, and comparing the results to a laboratory standard in order to correctly label each one of the COCs found per blood sample. A scatter plot (see Fig. 6K) shows the grouping of COCs from different samples into distinct clusters (cluster number is also shown). The average COAC of all reference blood samples is then determined and added to the scatter plot (see Fig. 6K). The assignment of cluster number to WBC class is achieved by measuring purposely enriched cell samples of only one cell type and checking the class assignment with a laboratory reference. Next, a Class Map with logical boundaries is created by either a) using data clustering techniques or b) manually via empirical observation (see Fig. 6L). The result is a labelled Class Map as shown in Fig. 6M.

This map can now be used on the user side via small adaptations to ensure it is applicable to the current data. This is achieved by a) using an empirically pre-determined reference class labeled map 12ao (Fig. 6M) in the form of a list of logical boundary edge coordinates in color-space for each of the three WBC type classes, accompanied by the average COAC of the reference class map, and b) a coordinate list of all COCs and accompanied by a list, with an entry for each coordinate, containing the k-means assigned cluster number; and also accompanied by the coordinate of the COAC of the user map (see Fig. 6O and 6P). To assign each cluster number to a class 12aq, the offset values between the coordinate position of the COAC of the user map and the coordinate position of the average COAC of the reference class map is calculated, followed by the independent application, for each corresponding element of the coordinates, of the offset values as a correction 12ap (by addition or subtraction) to a) each corresponding coordinate element of each logical boundary's edge coordinates list and b) to the coordinate of the average COAC of the reference map, in order to collocate the two COACs in color space (Fig. 6M and 6N) and generate an offset corrected edge coordinate list for each logical boundary. This is followed by an iterative check to determine to which WBC type class each associated cluster number belongs. This is achieved by successively thresholding/discriminating the COC coordinates against all the logical boundary's corrected edge coordinates (see Fig. 6O and 6P), derived for each WBC type class, with the aim of checking if a given COC is truly located in the logical region of the current WBC type class it is being checked against. This is shown in Fig. 6O and 6P and in tabular form in Fig. 7B.

As is shown in Fig. 6L it also would be possible to use the coordinate of the vertex (at which all three logical boundaries meet in color space) of the reference class map for adapting it to the user's color space map. This would be possible, since the average COAC of the reference map almost coincides with the vertex. However, this is not necessarily the case.

The final steps of the algorithm are to sum the populations of each cluster (Fig. 7A, 7B and 7C) assigned to a particular cell type class and repeat this for each cell type class 12ar. This results in a count per class for neutrophils 12as, lymphocytes 12at, and monocytes/others 12au. This is shown for two examples in Fig. 7C and Fig. 8C. Finally, the total cell count 12av is determined from the complete list of COTs 12ah. This total cell count is then used to calculate the percentage of the class population (count) for each cell type class 12aw, 12ax, and 12ay. The output for each class of the 3-part DCC algorithm is then given as 12az, 12ba, and 12bb.

A comparison with the laboratory DCC standard Sysmex Cell Counter (7-part) is shown for Example 1 and 2 in Fig. 7D and Fig. 8D. As can be seen, the 3-part DCC measurement method and algorithm described here has a small percentage error of less than 1.5% compared to the laboratory standard.

### Example 1. 3-part differential cell counting (DCC) in whole blood.

Sample preparation: A whole venous blood sample from a healthy volunteer was drawn with a NH₄-Heparin SARSTEDT Monovette^{®} and diluted 1:20 with Hank's Balanced Salt Solution (HBSS) at 37 °C. 10 µL of a stock solution of 10 µg/mL AO in HBSS was added to 90 µL of the diluted blood. 10 µL of the resulting sample was used for measurement.

The measurement was performed as described herein above with respect to Fig. 5 and 6. The measurement details were as follows:
- Camera settings: F 9.5, ISO 100;
- Exposure time: 1 sec;
- Imaging sequence: every 2 min, starting 1 min after addition of AO to sample;
- Number of total images: 21
- Total measurement time: 40 min
- Measurement conditions: Room temperature (about 25 °C)

As a reference measurement, the fresh blood sample was analyzed on a Sysmex cell counter XS-800i, based on light-scattering and fluorescence flow cytometry.

The measurement results are shown in Fig. 7. As can be seen from Fig. 7D, the inventive method gave an absolute error of less than 1.5 % compared to the laboratory standard for all three WBC classes.

### Example 2. 3-part differential cell counting (DCC) in whole blood.

A measurement was carried out in the same way as described in Example 1 using a further venous blood sample from another healthy volunteer.

The measurement results are shown in Fig. 8. In the same way as in Example 1, the inventive method gave an absolute error of less than 1.5 % compared to the laboratory standard for all three WBC classes (Fig. 8D).

### Example 3. 3-part differential cell counting (DCC) based on samples containing isolated or enriched white blood cells.

Isolated or enriched white blood cells are advantageous for the creation of a reference map in color space, which can be used for the classification of the cells and for the verification of the clustering methods used.

Preparation of samples with polymorphonuclear cells: Whole blood was drawn with NH₄-Heparin SARSTEDT Monovette^{®}. PMNs were separated with the procedure given by Lympholyte^{®}-poly Cell Separation Media (Cedarlane Technical protocol CL5070; www.cedar-lanelabs.com). In short: 5 mL of whole blood was layered over 5 ml of separation media, centrifuged at 1700 RPM for 30 min at 20 °C (Eppendorf centrifuge 5702 R). Two distinctive bands were formed. From the upper band mononuclear cells were drawn, from the lower band polymorphonuclear cells. Normal salinity was restored in the samples. The cells were washed, pelleted and diluted with HBSS before the addition of AO and measurements.

Preparation of samples with lymphocytes: Whole blood was drawn with NH₄-Heparin SARSTEDT Monovette^{®}. Lymphocytes were separated with the procedure given by Lympholyte^{®}-H Cell Separation Media. In short: 3 mL of whole blood were diluted with 3 mL of HBSS. This solution was layered over 3 mL of separation media, centrifuged at 2300 RPM for 20 min at 20 °C (Eppendorf centrifuge 5702 R). The resulting band was drawn off, and the cells were washed, pelleted and diluted with HBSS before the addition of AO and measurements.

Further proceeding and measurement conditions were identical to Examples 1 & 2.

## Claims

1. A method for classifying white blood cells (WBCs), the method comprising the steps of:
- providing a sample comprising a plurality of WBCs;
- adding at least one luminescent dye to the sample, thereby staining the WBCs;
- disposing the stained WBCs on a test surface;
- recording a luminescence image series comprising luminescence images of the stained WBCs on the test surface at a plurality of different timepoints, wherein each luminescence image comprises at least a first color channel and a second color channel;
- for each WBC of the plurality of WBCs, obtaining a luminescence data series by extracting from each luminescence image of the luminescence image series a luminescence datapoint associated with said WBC, wherein each luminescence datapoint comprises at least a first luminescence emission intensity value in the first color channel and a second luminescence emission intensity value in the second color channel; and
- analyzing the luminescence data series of each WBC in order to classify the WBC into one of at least two predetermined categories.

2. The method according to claim 1, wherein the luminescence image series comprises luminescence images at at least 4, preferably at least 10, different timepoints; wherein the different timepoints are separated from each other by at least 5 seconds, preferably at least 20 seconds.

3. The method according to claim 1 or 2, wherein each luminescence image further comprises a third color channel, and wherein each luminescence datapoint further comprises at least a third luminescence emission intensity value in the third color channel.

4. The method according to any one of claims 1 to 3, wherein the at least one luminescent dye is selected from the group consisting of acridine orange, hexidium iodide, SYBR 11, SYBR Green series dye, SYTO RNA Select, SYTO 11, SYTO 12, SYTO 13, SYTO 14, SYTO 16, SYTO 21, SYTO 24, and/or SYTO 25; preferably acridine orange.

5. The method according to any one of claims 1 to 4, wherein at least a first and a second luminescent dye are added to the sample, thereby staining the WBCs, preferably wherein the first and the second luminescent dye are each independently as defined in claim 4.

6. The method according to any one of claims 1 to 5, wherein analyzing the luminescence data series of each WBC comprises determining a trajectory over time of the WBC in a color space formed by said color channels.

7. The method according to claim 6, wherein analyzing the luminescence data series of each WBC further comprises determining a Centroid-Of-Trajectory (COT) for the WBC, wherein the COT corresponds to the centroid of the trajectory.

8. The method according to claim 7, wherein analyzing the luminescence data series of each WBC further comprises assigning the COT to an area of a predetermined color space map comprising a plurality of areas, each area corresponding to one of the pre-determined categories, thereby classifying the WBC into the category corresponding to said area.

9. The method according to claim 7 or 8, further comprising the step of clustering the COTs of the WBCs into a plurality of clusters, wherein the WBCs are classified by assigning each cluster to an area of the predetermined color space map, thereby classifying the WBCs contained in said cluster into the category corresponding to said area, preferably wherein the method further comprises the steps of determining for each cluster a Centroid-Of-Cluster (COC), wherein the COC corresponds to the centroid of the cluster; and optionally determining a Centroid-Of-All-Clusters (COAC), wherein the COAC corresponds to the centroid of the COCs; wherein the WBCs are classified by assigning each COC to an area of the predetermined color space map, thereby classifying the WBCs contained in the cluster corresponding to said COC into the category corresponding to said area.

10. The method according to any one of claims 8 or 9, further comprising the step of creating a calibrated color space map from the predetermined color space map, preferably by scaling the predetermined color space map to the COCs and/or by aligning the predetermined color space map with the COAC; wherein the WBCs are classified by
- assigning the COT to an area of the calibrated color space map, thereby classifying the WBC into the category corresponding to said area; and/or
- assigning each cluster to an area of the calibrated color space map, thereby classifying the WBCs contained in said cluster into the category corresponding to said area; and/or
- assigning each COC to an area of the calibrated color space map, thereby classifying the WBCs contained in the cluster corresponding to said COC into the category corresponding to said area.

11. The method according to any one of claims 1 to 10, wherein the at least two predetermined categories are selected from the group consisting of granulocytes, lymphocytes, and monocytes; and/or neutrophils, lymphocytes, monocytes, basophils, and eosinophils; preferably granulocytes and lymphocytes.

12. The method according to any one of the claims 1 to 11, wherein the WBCs are classified into one of at least three pre-determined categories, preferably granulocytes, lymphocytes, and monocytes; and/or at least three, preferably at least five, pre-determined categories selected from the group consisting of neutrophils, lymphocytes, monocytes, basophils, and eosinophils; especially neutrophils, lymphocytes and monocytes.

13. The method according to any one of the claims 1 to 12, further comprising the step of counting the number of WBCs in each of the predetermined categories and/or determining the proportion of WBCs in each of the predetermined categories; preferably, wherein the method is for obtaining a WBC differential, preferably a 3-part WBC differential or a 5-part WBC differential, especially a 3-part WBC differential.

14. An apparatus for classifying WBCs, preferably by the method according to any one of the preceding claims, the apparatus comprising:
- a sample holder (2) for holding a test surface comprising a plurality of luminescently stained WBCs;
- a luminescence imaging system (8a-d) suitable for recording from the test surface luminescence images comprising at least a first color channel and a second color channel; and
- a processor (8e-g) configured to carry out the following steps:
- obtaining a luminescence image series from the luminescence imaging system, the luminescence image series comprising luminescence images of the stained WBCs on the test surface at a plurality of different timepoints, wherein each luminescence image comprises at least a first color channel and a second color channel;
- for each WBC of the plurality of WBCs, obtaining a luminescence data series by extracting from each luminescence image of the luminescence image series a luminescence datapoint associated with said WBC, wherein each luminescence datapoint comprises at least a first luminescence emission intensity value in the first color channel and a second luminescence emission intensity value in the second color channel; and
- analyzing the luminescence data series of each WBC in order to classify the WBC into one of at least two predetermined categories.

15. The apparatus according to claim 14, wherein the processor is configured to carry out the steps as defined in any one of the claims 1 to 13.

## Patentansprüche

1. Ein Verfahren zur Klassifizierung von weißen Blutkörperchen (WBCs), wobei das Verfahren die folgenden Schritte aufweist:
- Bereitstellen einer Probe, die eine Mehrzahl von WBCs aufweist;
- Hinzufügen von mindestens einem Lumineszenzfarbstoff zu der Probe, wodurch die WBCs eingefärbt werden;
- Anordnen der eingefärbten WBCs auf einer Testoberfläche;
- Aufzeichnen einer Lumineszenz-Bildserie, die Lumineszenzbilder der eingefärbten WBCs auf der Testoberfläche zu einer Mehrzahl verschiedener Zeitpunkte aufweist, wobei jedes Lumineszenzbild mindestens einen ersten Farbkanal und einen zweiten Farbkanal aufweist;
- für jedes WBC der Mehrzahl von WBCs, Erhalten einer Lumineszenz-Datenserie durch Extrahieren eines Lumineszenz-Datenpunkts aus jedem Lumineszenzbild der Lumineszenz-Bildserie, die mit besagtem WBC assoziiert ist, wobei jeder Lumineszenz-Datenpunkt mindestens einen ersten Lumineszenz-Emissionsintensitätswert im ersten Farbkanal und einen zweiten Lumineszenz-Emissionsintensitätswert im zweiten Farbkanal aufweist; und
- Analysieren der Lumineszenz-Datenserie jedes WBC, um das WBC in eine von mindestens zwei vorbestimmten Kategorien zu klassifizieren.

2. Das Verfahren nach Anspruch 1, wobei die Lumineszenz-Bildserie Lumineszenzbilder zu mindestens 4, vorzugsweise mindestens 10, verschiedenen Zeitpunkten aufweist, wobei die verschiedenen Zeitpunkte durch mindestens 5 Sekunden, vorzugsweise mindestens 20 Sekunden, voneinander getrennt sind.

3. Das Verfahren nach Anspruch 1 oder 2, wobei jedes Lumineszenzbild ferner einen dritten Farbkanal aufweist, und wobei jeder Lumineszenz-Datenpunkt ferner mindestens einen dritten Lumineszenz-Emissionsintensitätswert im dritten Farbkanal aufweist.

4. Das Verfahren nach einem der Ansprüche 1 bis 3, wobei der mindestens eine Lumineszenzfarbstoff aus der Gruppe ausgewählt ist, die aus Acridinorange, Hexidiumiodid, SYBR 11, Farbstoffe der SYBR Green-Serie, SYTO RNA Select, SYTO 11, SYTO 12, SYTO 13, SYTO 14, SYTO 16, SYTO 21, SYTO 24 und/oder SYTO 25 besteht, vorzugsweise Acridinorange.

5. Das Verfahren nach einem der Ansprüche 1 bis 4, wobei mindestens ein erster und ein zweiter Lumineszenzfarbstoff zu der Probe hinzugefügt werden, wodurch die WBCs eingefärbt werden, vorzugsweise wobei der erste und der zweite Lumineszenzfarbstoff jeweils unabhängig voneinander wie in Anspruch 4 definiert ist.

6. Das Verfahren nach einem der Ansprüche 1 bis 5, wobei das Analysieren der Lumineszenz-Datenserie jedes WBC das Bestimmen einer zeitlichen Trajektorie des WBC in einem durch die Farbkanäle gebildeten Farbraum aufweist.

7. Das Verfahren nach Anspruch 6, wobei das Analysieren der Lumineszenz-Datenserie jedes WBC ferner das Bestimmen eines Centroid-Of-Trajectory (COT) für das WBC aufweist, wobei der COT dem Zentroid der Trajektorie entspricht.

8. Das Verfahren nach Anspruch 7, wobei das Analysieren der Lumineszenz-Datenserie jedes WBC ferner das Zuordnen des COT zu einem Bereich einer vorbestimmten Farbraumkarte aufweist, die eine Mehrzahl von Bereichen aufweist, wobei jeder Bereich einer der vorbestimmten Kategorien entspricht, wodurch das WBC in die Kategorie klassifiziert wird, die dem Bereich entspricht.

9. Das Verfahren gemäß Anspruch 7 oder 8, das ferner den Schritt des Clusterns der COTs der WBCs in eine Mehrzahl von Clustern aufweist, wobei die WBCs klassifiziert werden, indem jeder Cluster einem Bereich der vorbestimmten Farbraumkarte zugeordnet wird, wodurch die in diesem Cluster enthaltenen WBCs in die diesem Bereich entsprechende Kategorie klassifiziert werden, wobei das Verfahren vorzugsweise ferner die folgenden Schritte aufweist: Bestimmen eines Centroid-Of-Cluster (COC) für jeden Cluster, wobei das COC dem Zentroid des Clusters entspricht; und optional das Bestimmen eines Centroid-Of-All-Clusters (COAC), wobei der COAC dem Zentroid der COCs entspricht; wobei die WBCs klassifiziert werden, indem jeder COC einem Bereich der vorbestimmten Farbraumkarte zugeordnet wird, wodurch die in dem Cluster enthaltenen WBCs, die dem COC entsprechen, in die Kategorie klassifiziert werden, die dem Bereich entspricht.

10. Das Verfahren nach einem der Ansprüche 8 oder 9, ferner aufweisend den Schritt des Erstellens einer kalibrierten Farbraumkarte aus der vorbestimmten Farbraumkarte, vorzugsweise durch Skalieren der vorbestimmten Farbraumkarte auf die COCs und/oder durch Ausrichten der vorbestimmten Farbraumkarte am COAC; wobei die WBCs klassifiziert werden durch
- Zuweisen des COT zu einem Bereich der kalibrierten Farbraumkarte, wodurch das WBC in die Kategorie klassifiziert wird, die besagtem Bereich entspricht; und/oder
- Zuweisen jedes Clusters zu einem Bereich der kalibrierten Farbraumkarte, wodurch die WBCs, die in besagtem Cluster enthalten sind, in die Kategorie klassifiziert werden, die besagtem Bereich entspricht; und/oder
- Zuweisen jedes COC zu einem Bereich der kalibrierten Farbraumkarte, wodurch die WBCs, die in dem Cluster enthalten sind, das besagtem COC entspricht, in die Kategorie klassifiziert werden, die besagtem Bereich entspricht.

11. Das Verfahren gemäß einem der Ansprüche 1 bis 10, wobei die mindestens zwei vorbestimmten Kategorien aus der Gruppe bestehend aus Granulozyten, Lymphozyten und Monozyten; und/oder Neutrophilen, Lymphozyten, Monozyten, Basophilen und Eosinophilen; vorzugsweise Granulozyten und Lymphozyten, ausgewählt sind.

12. Das Verfahren nach einem der Ansprüche 1 bis 11, wobei die WBCs in eine von mindestens drei vorbestimmten Kategorien, vorzugsweise Granulozyten, Lymphozyten und Monozyten, und/oder mindestens drei, vorzugsweise mindestens fünf, vorbestimmten Kategorien, ausgewählt aus der Gruppe bestehend aus Neutrophilen, Lymphozyten, Monozyten, Basophilen und Eosinophilen, insbesondere Neutrophilen, Lymphozyten und Monozyten, klassifiziert werden.

13. Das Verfahren nach einem der Ansprüche 1 bis 12, das ferner den Schritt des Zählens der Anzahl der WBCs in jeder der vorbestimmten Kategorien und/oder des Bestimmens des Anteils der WBCs in jeder der vorbestimmten Kategorien aufweist; vorzugsweise wobei das Verfahren zum Erhalten eines WBC-Differentialblutbilds, vorzugsweise eines 3-teiligen WBC-Differentialblutbilds oder eines 5-teiligen WBC-Differentialblutbilds, insbesondere eines 3-teiligen WBC-Differentialblutbilds, dient.

14. Eine Vorrichtung zum Klassifizieren von WBCs, vorzugsweise nach dem Verfahren gemäß einem der vorstehenden Ansprüche, wobei die Vorrichtung folgendes aufweist:
- einen Probenhalter zum Halten einer Testoberfläche, die eine Mehrzahl von lumineszenzgefärbten WBCs aufweist;
- ein Lumineszenz-Bildgebungssystem (8a-d), das zur Aufzeichnung von Lumineszenzbildern von der Testoberfläche geeignet ist und mindestens einen ersten Farbkanal und einen zweiten Farbkanal aufweist; und
- einen Prozessor (8e-g), der so konfiguriert ist, dass er die folgenden Schritte ausführt:
- Erhalten einer Lumineszenz-Bildserie aus dem Lumineszenzbildgebungssystem, wobei die Lumineszenz-Bildserie Lumineszenzbilder der eingefärbten WBCs auf der Testoberfläche zu mehreren verschiedenen Zeitpunkten aufweist, wobei jedes Lumineszenzbild mindestens einen ersten Farbkanal und einen zweiten Farbkanal aufweist;
- für jedes WBC der Mehrzahl von WBCs Erhalten einer Lumineszenz-Datenserie durch Extrahieren eines Lumineszenz-Datenpunkts, der mit dem WBC assoziiert ist, aus jedem Lumineszenzbild der Lumineszenz-Bildserie, wobei jeder Lumineszenz-Datenpunkt mindestens einen ersten Lumineszenz-Emissionsintensitätswert im ersten Farbkanal und einen zweiten Lumineszenz-Emissionsintensitätswert im zweiten Farbkanal aufweist; und
- Analysieren der Lumineszenz-Datenserie jedes WBC, um den WBC in eine von mindestens zwei vorbestimmten Kategorien zu klassifizieren.

15. Die Vorrichtung nach Anspruch 14, wobei der Prozessor so konfiguriert ist, dass er die in einem der Ansprüche 1 bis 13 definierten Schritte ausführt.

## Revendications

1. Procédé de classification des globules blancs (GB), le procédé comprenant les étapes consistant à:
- fournir un échantillon comprenant une pluralité de GB;
- ajouter au moins un colorant luminescent à l'échantillon, colorant ainsi les GB;
- disposer les GB colorés sur une surface de test;
- enregistrer une série d'images de luminescence comprenant des images de luminescence des GB colorés sur la surface de test à une pluralité de points temporels différents, chaque image de luminescence comprenant au moins un premier canal de couleur et un second canal de couleur;
- pour chaque GB de la pluralité de GB, obtenir une série de données de luminescence en extrayant de chaque image de luminescence de la série d'images de luminescence un point de données de luminescence associé audit GB, chaque point de données de luminescence comprenant au moins une première valeur d'intensité d'émission de luminescence dans le premier canal de couleur et une seconde valeur d'intensité d'émission de luminescence dans le second canal de couleur; et
- analyser la série de données de luminescence de chaque GB afin de classer le GB dans l'une d'au moins deux catégories prédéterminées.

2. Le procédé selon la revendication 1, dans lequel la série d'images de luminescence comprend des images de luminescence à au moins 4, de préférence au moins 10, points temporels différents; dans lequel les différents points temporels sont séparés les uns des autres par au moins 5 secondes, de préférence au moins 20 secondes.

3. Le procédé selon la revendication 1 ou 2, dans lequel chaque image de luminescence comprend en outre un troisième canal de couleur, et dans lequel chaque point de données de luminescence comprend en outre au moins une troisième valeur d'intensité d'émission de luminescence dans le troisième canal de couleur.

4. Le procédé selon l'une des revendications 1 à 3, dans lequel le au moins un colorant luminescent est choisi dans le groupe constitué par l'acridine orange, l'iodure d'hexidium, SYBR 11, le colorant de la série SYBR Green, SYTO RNA Select, SYTO 11, SYTO 12, SYTO 13, SYTO 14, SYTO 16, SYTO 21, SYTO 24, et/ou SYTO 25; de préférence l'acridine orange.

5. Le procédé selon l'une des revendications 1 à 4, dans lequel au moins un premier et un second colorant luminescent sont ajoutés à l'échantillon, colorant ainsi les GB, de préférence dans lequel le premier et le second colorant luminescent sont chacun indépendamment tels que définis dans la revendication 4.

6. Le procédé selon l'une des revendications 1 à 5, dans lequel l'analyse de la série de données de luminescence de chaque GB comprend la détermination d'une trajectoire au fil du temps du GB dans un espace colorimétrique formé par lesdits canaux de couleur.

7. Le procédé selon la revendication 6, dans lequel l'analyse de la série de données de luminescence de chaque GB comprend en outre la détermination d'un Centroïde-De-Trajectoire (CDT) pour le GB, le CDT correspondant au centroïde de la trajectoire.

8. Le procédé selon la revendication 7, dans lequel l'analyse de la série de données de luminescence de chaque GB comprend en outre l'attribution du CDT à une zone d'une carte d'espace colorimétrique prédéterminée comprenant une pluralité de zones, chaque zone correspondant à l'une des catégories prédéterminées, classant ainsi le GB dans la catégorie correspondant à ladite zone.

9. Le procédé selon la revendication 7 ou 8, comprenant en outre l'étape de regroupement des CDT des GB en une pluralité de groupes, dans lequel les GB sont classés en attribuant chaque groupe à une zone de la carte d'espace colorimétrique prédéterminée, classant ainsi les GB contenus dans ledit groupe dans la catégorie correspondant à ladite zone, de préférence dans lequel le procédé comprend en outre les étapes de détermination pour chaque groupe d'un Centroïde-De-Groupe (CDG), le CDG correspondant au centroïde du groupe; et éventuellement de détermination d'un Centroïde-De-Tous-Les-Groupes (CDTG), le CDTG correspondant au centroïde des CDG; dans lequel les GB sont classés en attribuant chaque CDG à une zone de la carte d'espace colorimétrique prédéterminée, classant ainsi les GB contenus dans le groupe correspondant audit CDG dans la catégorie correspondant à ladite zone.

10. Le procédé selon l'une des revendications 8 ou 9, comprenant en outre l'étape de création d'une carte d'espace colorimétrique calibrée à partir de la carte d'espace colorimétrique prédéterminée, de préférence par mise à l'échelle de la carte d'espace colorimétrique prédéterminée aux CDG et/ou par alignement de la carte d'espace colorimétrique prédéterminée avec le CDTG; dans lequel les GB sont classés en
- attribuant le CDT à une zone de la carte d'espace colorimétrique calibrée, classant ainsi le GB dans la catégorie correspondant à ladite zone; et/ou
- attribuant chaque groupe à une zone de la carte d'espace colorimétrique calibrée, classant ainsi les GB contenus dans ledit groupe dans la catégorie correspondant à ladite zone; et/ou
- attribuant chaque CDG à une zone de la carte d'espace colorimétrique calibrée, classant ainsi les GB contenus dans le groupe correspondant audit CDG dans la catégorie correspondant à ladite zone.

11. Le procédé selon l'une des revendications 1 à 10, dans lequel les au moins deux catégories prédéterminées sont choisies dans le groupe constitué par les granulocytes, les lymphocytes et les monocytes; et/ou les neutrophiles, les lymphocytes, les monocytes, les basophiles et les éosinophiles; de préférence les granulocytes et les lymphocytes.

12. Le procédé selon l'une des revendications 1 à 11, dans lequel les GB sont classés dans l'une d'au moins trois catégories prédéterminées, de préférence les granulocytes, les lymphocytes et les monocytes; et/ou au moins trois, de préférence au moins cinq, catégories prédéterminées choisies dans le groupe constitué par les neutrophiles, les lymphocytes, les monocytes, les basophiles et les éosinophiles; en particulier les neutrophiles, les lymphocytes et les monocytes.

13. Le procédé selon l'unedes revendications 1 à 12, comprenant en outre l'étape de comptage du nombre de GB dans chacune des catégories prédéterminées et/ou de détermination de la proportion de GB dans chacune des catégories prédéterminées; de préférence, dans lequel le procédé est destiné à obtenir une formule leucocytaire, de préférence une formule leucocytaire à 3 populations ou une formule leucocytaire à 5 populations, en particulier une formule leucocytaire à 3 populations.

14. Appareil pour classer les GB, de préférence par le procédé selon l'une des revendications précédentes, l'appareil comprenant:
- un porte-échantillon (2) pour maintenir une surface de test comprenant une pluralité de GB colorés par luminescence;
- un système d'imagerie de luminescence (8a-d) adapté pour enregistrer à partir de la surface de test des images de luminescence comprenant au moins un premier canal de couleur et un second canal de couleur; et
- un processeur (8e-g) configuré pour exécuter les étapes suivantes:
- obtenir une série d'images de luminescence à partir du système d'imagerie de luminescence, la série d'images de luminescence comprenant des images de luminescence des GB colorés sur la surface de test à une pluralité de points temporels différents, chaque image de luminescence comprenant au moins un premier canal de couleur et un second canal de couleur;
- pour chaque GB de la pluralité de GB, obtenir une série de données de luminescence en extrayant de chaque image de luminescence de la série d'images de luminescence un point de données de luminescence associé audit GB, chaque point de données de luminescence comprenant au moins une première valeur d'intensité d'émission de luminescence dans le premier canal de couleur et une seconde valeur d'intensité d'émission de luminescence dans le second canal de couleur; et
- analyser la série de données de luminescence de chaque GB afin de classer le GB dans l'une d'au moins deux catégories prédéterminées.

15. L'appareil selon la revendication 14, dans lequel le processeur est configuré pour exécuter les étapes telles que définies dans l'une des revendications 1 à 13.
